# EUROPEAN PATENT SPECIFICATION

(11) **EP 3 884 047 B1**
(45) Date of publication and mention of the grant of the patent: **08.10.2025**
(21) Application number: 19886590.9
(22) Date of filing: 20.11.2019
(51) Int. Cl.: C12Q 1/6806, C12N 15/10, C12Q 1/6827

(54) **METHODS FOR TARGETED NUCLEIC ACID LIBRARY FORMATION**
VERFAHREN ZUR GEZIELTEN BILDUNG VON NUKLEINSÄUREBIBLIOTHEKEN
MÉTHODES DE FORMATION DE BIBLIOTHÈQUE D'ACIDES NUCLÉIQUES CIBLÉE

(30) Priority: 21.11.2018 US 201862770585 P
(43) Date of publication of application: 29.09.2021
(60) Divisional application: 25199249.1
(73) Proprietor: Agilent Technologies, Inc., Santa Clara CA 95051 (US)
(72) Inventor: LIN, Shengrong, Fremont, California 94555 (US); ZHAO, Grace, Palo Alto, California 94306 (US); BAO, Yun, Fremont, California 94539 (US); WANG, Heng, Pleasanton, California 94566 (US)
(74) Representative: Grünecker Patent- und Rechtsanwälte PartG mbB
(86) International application number: PCT/US2019/062507
(87) International publication number: WO 2020/106906

(56) References cited:
- WO-A2-01/57268
- CN-A- 103 806 111
- US-A1- 2012 053 087
- US-A1- 2015 011 396
- US-A1- 2016 002 720
- US-A1- 2016 215 331
- US-A1- 2017 067 117
- US-A1- 2017 226 575
- US-A1- 2018 066 306
- US-A1- 2018 291 371
- US-A1- 2018 320 241
- KOLPASHCHIKOV D M: "A BINARY DNA PROBE FOR HIGHLY SPECIFIC NUCLEIC ACID RECOGNITION", JOURNAL OF THE AMERICAN CHEMICAL SOCIETY, AMERICAN CHEMICAL SOCIETY, vol. 128, no. 32, 16 August 2006 (2006-08-16), pages 10625 - 10628, XP008130557, ISSN: 0002-7863, [retrieved on 20060721], DOI: 10.1021/JA0628093
- CORNETT EVAN M. ET AL: "Operating Cooperatively (OC) Sensor for Highly Specific Recognition of Nucleic Acids", PLOS ONE, vol. 8, no. 2, 1 February 2013 (2013-02-01), pages e55919, XP055938723, DOI: 10.1371/journal.pone.0055919
- WANG FAY ET AL: "RNAscope A Novel in Situ RNA Analysis Platform for Formalin-Fixed, Paraffin-Embedded Tissues", J MOL DIAGN, 1 January 2012 (2012-01-01), pages 22 - 2908002, XP055938735, Retrieved from the Internet <URL:https://www.ncbi.nlm.nih.gov/pmc/articles/PMC3338343/pdf/main.pdf> [retrieved on 20220705]
- KAIXIANG ZHANG ET AL: "Direct Visualization of Single-Nucleotide Variation in mtDNA Using a CRISPR/Cas9-Mediated Proximity Ligation Assay", JOURNAL OF THE AMERICAN CHEMICAL SOCIETY, vol. 140, no. 36, 12 September 2018 (2018-09-12), pages 11293 - 11301, XP055552112, ISSN: 0002-7863, DOI: 10.1021/jacs.8b05309
- BRIGGS ADRIAN W ET AL: "Patterns of damage in genomic DNA sequences from a Neandertal", PROCEEDINGS OF THE NATIONAL ACADEMY OF SCIENCES, NATIONAL ACADEMY OF SCIENCES, vol. 104, no. 37, 11 September 2007 (2007-09-11), pages 14616 - 14621, XP002523498, ISSN: 0027-8424, DOI: 10.1073/PNAS.0704665104
- PEETERS S ET AL: "Specific magnetic isolation for direct detection of HPV16", EUROPEAN JOURNAL OF CLINICAL MICROBIOLOGY & INFECTIOUS DISEASES, SPRINGER, BERLIN, DE, vol. 31, no. 4, 2 August 2011 (2011-08-02), pages 539 - 546, XP035029076, ISSN: 1435-4373, DOI: 10.1007/S10096-011-1345-4
- YEUNG S W ET AL: "Manipulation and extraction of genomic DNA from cell lysate by functionalized magnetic particles for lab on a chip applications", BIOSENSORS AND BIOELECTRONICS, ELSEVIER SCIENCE LTD, UK, AMSTERDAM , NL, vol. 21, no. 7, 15 January 2006 (2006-01-15), pages 989 - 997, XP024961376, ISSN: 0956-5663, [retrieved on 20060115], DOI: 10.1016/J.BIOS.2005.03.008

## Description

### CROSS-REFERENCE

This application claims priority to U.S. Provisional Application No. 62/770,585, filed on November 21, 2018.

### BACKGROUND

Nucleic acid target capture methods can allow specific genes, exons, and other genomic regions of interest to be enriched, e.g., for targeted sequencing. However, the current target capture-based sequencing methods can involve cumbersome lengthy protocols and costly processes. Current target capture-based methods can also have a low on-target rate for a small capture panel (e.g., less than 500 probes) and low flexibility for multiplex-PCR amplicon-based target sequencing due, e.g., to primer interaction, especially for PCR primers with molecular barcodes. Moreover, current methods for nucleic acid target capture can be ill-suited for low input and damaged DNA because of a low conversion rate.

Bisulfite conversion can be a useful technique to study the methylation pattern of nucleic acid molecules and can be used to study methylation by sequencing. However, bisulfite conversion can damage nucleic acids by creating truncations for example. If a next-generation sequencing (NGS) DNA library is treated with bisulfite, a substantial amount of the nucleic acids can be damaged and be unable to be recovered in the subsequent amplification steps, and thereby provide a low conversation rate. Moreover, because the bisulfite conversion can result in single stranded or fragmented DNA and reduced sequence complexity, converted DNA can be a difficult input for conventional adaptor-ligation based library construction. Bisulfite treated cell-free (cfDNA) or circulating tumor cell DNA (ctDNA) with typically small initial input can present a bigger challenge given the low conversion rate (e.g. 5% or less for bisulfite treated cfDNA). In addition, upon bisulfite conversion, any non-methylated "C" can be converted to "U", which in turn can be converted to "T" after PCR amplification. Since the pre-bisulfite conversion amplification may not preserve the methylation information, post-bisulfite conversion capture can be more commonly used. The probe or PCR primer design or production can be based on the post-bisulfite converted sequences, and it is a more complicated process. If a pre-bisulfite conversion capture is used, the existing market protocol may require high DNA input ( e.g. more than 250ng). Such constraints can cause the targeted regions to be difficult to enrich by current probe capture or multiplexed-PCR approaches.

Therefore, there is a need for a more efficient, easy to use, fast, flexible, and practical target nucleic acid capture methods and improved methods for analyzing bisulfite treated nucleic acid especially for the low-input samples such as cfDNA. The method disclosed herein can be used for pre-amplification and pre-bisulfite conversion hybridization-based capture for a very low DNA input samples.

US 2018/0066306 A1 relates to methods for the enrichment and analysis of target nucleic acid from a biological sample, for example, by using two capture probes to connect the target nucleic acid molecule to a ligation probe through hybridization. Briggs et al. (Briggs A.W. et al. 2007: Patterns of damage in genomic DNA sequences from a Neandertal, PNAS 104(37): 14616-14621) relates to methods of preparing a library of a target nucleic acid molecule for sequencing by using two adapters.

### SUMMARY

The present invention is defined in the claims.

The present invention relates to a method comprising (a) hybridizing a first target specific region of a first bridge probe to a first target sequence of a template nucleic acid molecule, wherein the first bridge probe comprises an adaptor sequence positioned 5' of the target specific region and hybridizing a first landing sequence of the first bridge probe to a first bridge binding sequence of an anchor probe; (b) hybridizing a second target specific region of a second bridge probe to a second target sequence of the template nucleic acid molecule, and hybridizing a second landing sequence of the second bridge probe to a second bridge binding sequence of the anchor probe, thereby forming a complex comprising the template nucleic acid molecule, the first bridge probe, the second bridge probe, and the anchor probe, wherein the anchor probe is not attached to a solid support during (a) and (b); (c) following (a) and (b), coupling the anchor probe to the solid support; and (d) following (c), contacting the complex with a 3' to 5' exonuclease, wherein the 3' to 5' exonuclease cleaves the 3' end of the template nucleic acid molecule and extending the 3' end of the template nucleic acid molecule using the adaptor sequence as a template, thereby generating a first extension product.

The anchor probe may further comprise a binding moiety, and the coupling may further comprise attaching the binding moiety to the solid support. The solid support may be a bead. The bead may be a streptavidin bead. The binding moiety may be a biotin. The template nucleic acid molecule may be cell-free DNA. The method may further comprise contacting the first extension product with a primer comprising a sequence of the adaptor to generate a second extension product. The method may further comprise i) hybridizing a target specific primer to the second extension product and extending the target specific primer to generate a third extension product or ii) attaching a second double-stranded adaptor to the 5' end of the first extension product and the 3' end of the second extension product. The method may further comprise detecting a methylation pattern of the template nucleic acid molecule. The method may further comprise analyzing the amplified nucleic acid product for one or more nucleic acid features of the template nucleic acid molecule selected from the group of the following features: a de novo mutation, nonsense mutation, missense mutation, silent mutation, frameshift mutation, insertion, substitution, point mutation, single nucleotide polymorphism (SNP), single nucleotide variant (SNV), de novo single nucleotide variant, deletion, rearrangement, amplification, chromosomal translocation, interstitial deletion, chromosomal inversion, loss of heterozygosity, loss of function, gain of function, dominant negative, or lethal mutation.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

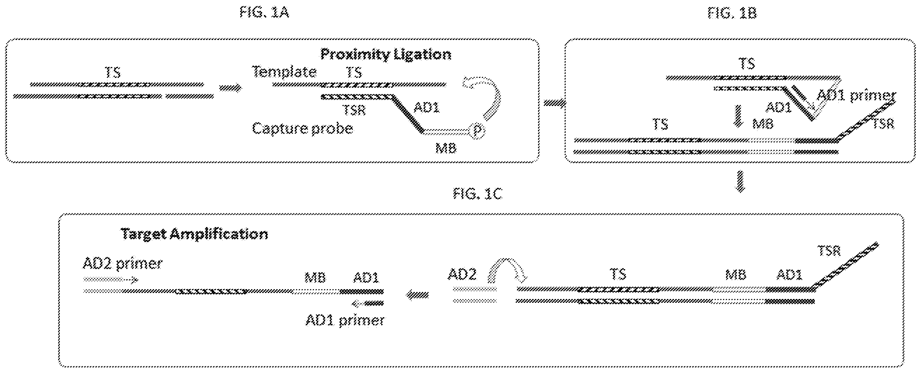

**FIGS. 1A-1C** illustrate one embodiment of direct hybridization of a capture probe to a target sequence (TS) in a template and subsequent amplification. **FIG. 1A** shows hybridization of the capture probe to the TS and proximity ligation of the capture probe to the template. **FIG. 1B** shows synthesis of a complementary strand to the template using an AD1 primer. **FIG. 1C** illustrates ligation of a second adaptor (AD2) to the extension product and template from. **FIG. 1B** and a primer that anneals to the second adaptor for further amplification of the TS using AD2 primer and AD1 primer.
**FIGS. 2A-2C** illustrate one embodiment of direct hybridization of a capture probe to a target sequence (TS) in a template and a different amplification method. **FIG. 2A** shows hybridization of the capture probe to the target sequence and proximity ligation of the capture probe to the template. **FIG. 2B** shows synthesis of a complementary strand to the template using AD1 primer. **FIG. 2C** illustrates the use of a target specific region (TSR) primer for further amplification of the target sequence with the AD1 primer.

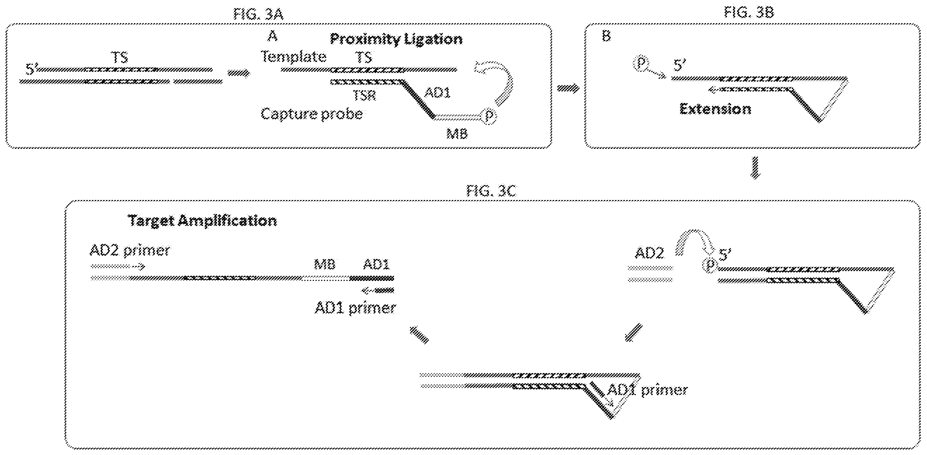

**FIGS. 3A-3C** show an embodiment of a direct hybridization of a capture probe to a target sequence (TS) and another amplification method. **FIG. 3A** shows hybridization of the capture probe to the target sequence and proximity ligation of the capture probe to the template. **FIG. 3B** shows phosphorylation of the 5' end of the template and synthesis of a complementary strand to the template using the target specific region (TSR) of the capture probe (3' end of the capture probe) as a primer. **FIG. 3C** illustrates ligation of AD2 adaptor to the product of FIG. 3B and the use of the primers to anneal to AD1 and AD2 for further amplification of the target sequence.
**FIGS. 4A-4C** illustrate the removal of a target specific region (TSR) of a capture probe to facilitate subsequent amplification of a target sequence. **FIG. 4A** shows hybridization of the capture probe to the target sequence of a template and proximity ligation of the capture probe to the template. **FIG. 4B** shows cleavage of the TSR of the capture probe. **FIG. 4C** shows extension of the AD1 primer annealed to the AD1 sequence in the capture probe and amplification of the target sequence.

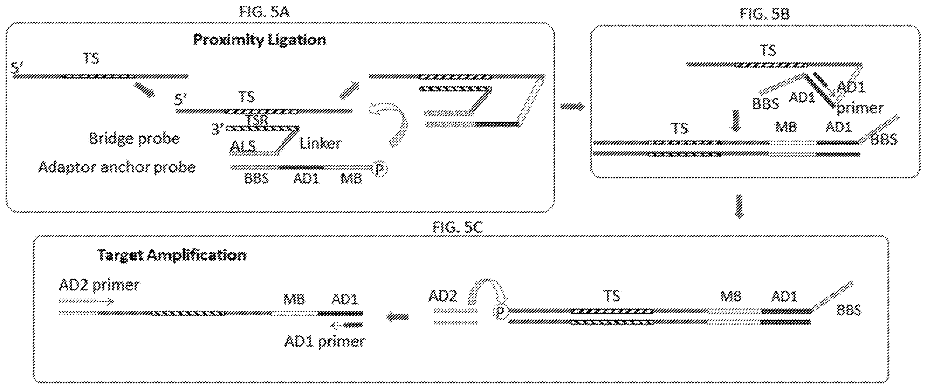

**FIGS. 5A-5C** show indirect association of an adaptor anchor probe to the template. **FIG.** 5A shows the association of adaptor anchor probe with the template due to hybridization of a bridge probe to both the adaptor anchor probe and the template and subsequent ligation of the adaptor anchor probe to the template. The target-specific region (TSR) is located at the 3'-portion of the bridge probe. **FIG. 5B** shows synthesis of a complementary strand to the template using an AD1 primer. **FIG. 5C** shows ligation of an AD2 adaptor to the product of FIG. 5B and further amplification using AD1 and AD2 primers.

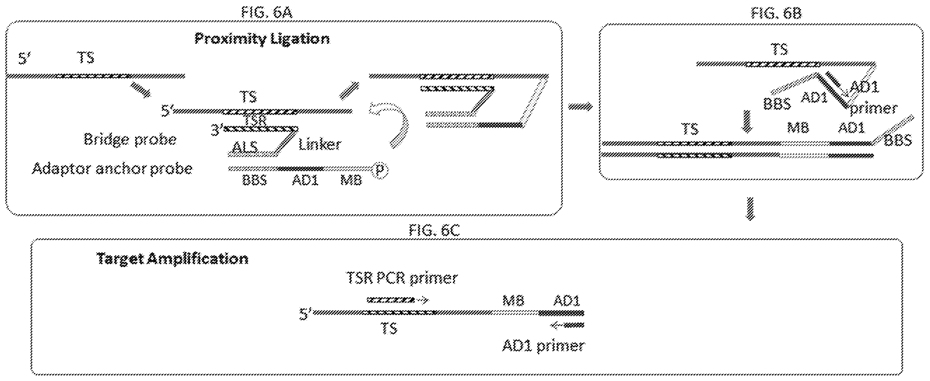

**FIGS. 6A-6C** show indirect association of an adaptor anchor probe and a different amplification method. **FIG. 6A** shows the association of adaptor anchor probe with the template due to hybridization of bridge probe to both adaptor anchor probe and the template and subsequent ligation of the adaptor anchor probe to the template. The target-specific region (TSR) is located at the 3'-portion of the bridge probe. **FIG. 6B** shows synthesis of a complementary strand to the template using AD1 primer. **FIG. 6C** shows further amplification using a primer that anneals to the complement of the target sequence and the AD1 primer.

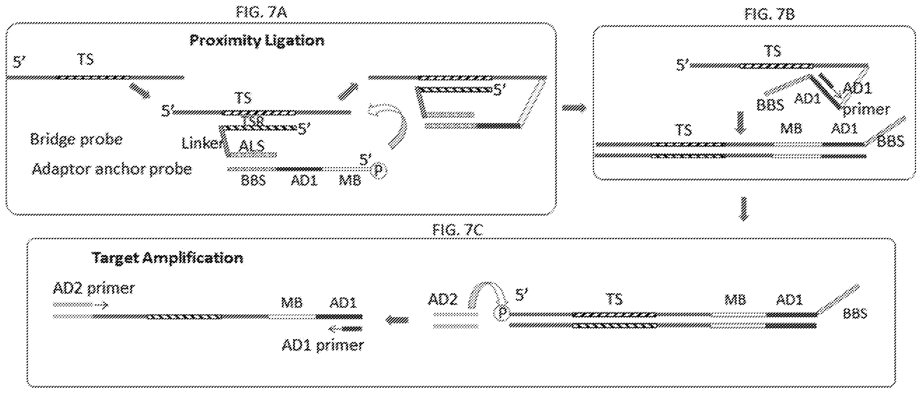

**FIGS. 7A-7C** show indirect association of an adaptor anchor probe to a template using a bridge probe wherein the target specific region (TSR) is located at the 5'-portion of the bridge probe. **FIG. 7A** shows the association of adaptor anchor probe with the template due to hybridization of bridge probe to both adaptor anchor probe and the template and subsequent ligation of the adaptor anchor probe to the template. **FIG. 7B** shows synthesis of a complementary strand to the template using AD1 primer. The bonds between the bridge probe, the template, and the adaptor anchor probe can be denatured before annealing a primer to the AD1 sequence. **FIG. 7C** shows ligation of AD2 adaptor to the product of FIG. 7B and further amplification using AD1 and AD2 primers.

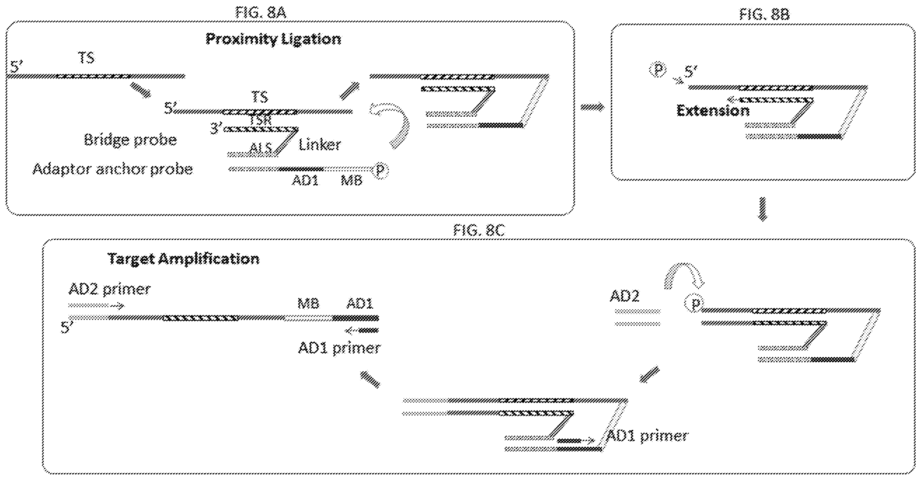

**FIGS. 8A-8C** show indirect association of an adaptor anchor probe to a template via a bridge probe and extension of a target specific region (TSR) of the bridge probe. **FIG. 8A** shows the association of the adaptor anchor probe with the template due to hybridization of bridge probe to both the adaptor anchor probe and the template and subsequent ligation of the adaptor anchor probe to the template. **FIG. 8B** shows synthesis of a complementary strand to the template using TSR as a primer (3' end of the bridge probe). **FIG. 8C** shows ligation of the AD2 adaptor to the product of **FIG. 8B** and further amplification using AD1 and AD2 primers.
**FIG. 9** shows hybridization of multiple bridge probes to a template, and to an adaptor anchor probe, leading to proximity ligation of the adaptor anchor probe to the template. Both bridge probes comprise a target specific region (TSR) at a 3'-portion.
**FIG. 10** shows hybridization of another set of bridge probes to a template, and to an adaptor anchor probe, and subsequent ligation of adaptor anchor probe to the template. Both bridge probes comprise a target specific region (TSR) at a 5'-portion.
**FIG. 11** shows hybridization of different bridge probes to a template and ligation of an adaptor anchor probe to the template. The first bridge probe comprises a target specific region (TSR) at the 3'-portion and the second bridge probe comprises the TSR at the 5'-portion, leading to hybridization of linkers of both bridge probes and a stable assembly.
**FIG. 12** shows hybridization of bridge probes to a template and ligation of an adaptor anchor probe to the template. The first bridge probe comprises the TSR at the 5'-portion and the second bridge probe comprises the TSR at the 3'-portion.
**FIGS. 13A-B** illustrate two embodiments for target amplification with different orders of bisulfite treatment with respect to the probe ligation to a template comprising the target. **FIG. 13A** shows bisulfite treatment of DNA Input prior to its hybridization to capture probes or bridge probes. **FIG. 13B** shows bisulfite treatment of DNA Input after its hybridization and proximity ligation to capture probes or bridge probes.
**FIG. 14** shows an effect of bisulfite treatment on ligated template DNA. Bisulfite conversion can leave the double stranded portion of template DNA intact, allowing the use of TSR PCR primer designed to anneal to the complement of non-bisulfite converted TS of the template DNA.
**FIGS. 15A-15B** show a scheme for selective removal of an off-target ligation product using exonuclease.
**FIGS. 16A-16B** shows solid-phase capture methods for selection of properly ligated target template and adaptor anchor probe.
**FIGS. 17A-17C** show a library construction method using either ssDNA or damaged dsDNA as starting material.
**FIG. 18A** illustrates a schematic for attaching an adaptor anchor probe to a template with (1) or without (2) a bridge probe. **FIG. 18B** shows an image of an agarose gel with ligated template and adaptor anchor probe products for the set up with a bridge probe(1); lack of ligation product for the set up without a bridge probe (2).
**FIGS. 19A-19C** illustrate different methods of adaptor addition after direct hybridization of the capture probe to template nucleic acid.
**FIGS. 20A-20D** show different methods of adaptor addition after indirect association of the template nucleic acid with adaptor anchor probe through interaction with bridge probe.
**FIGS. 21A-21E** show various configurations of indirect association of the template nucleic acid and adaptor anchor probe through interaction with bridge probe.
**FIGS. 22A-22G** illustrate the steps for adaptor addition through direct hybridization of the template nucleic acid with the target probe, and digestion of the template nucleic acid by 3' to 5' exonuclease.
**FIGS. 23A-23F** illustrate the steps for adaptor addition through indirect association of the target probe and the template nucleic acid, and extension of the bridge probe.
**FIGS. 24A-24F** illustrate the steps for adaptor addition through indirect association of the target probe and the template nucleic acid, and digestion of the template nucleic acid by 3' to 5' exonuclease.
**FIGS. 25A-25D** illustrate the steps for adaptor addition through the use of polynucleotide primers with adaptors.
**FIG. 26** shows a schematic overview of an experiment for adaptor addition by direct hybridization by capture probe and digestion by 3' to 5' exonuclease of template nucleic acid using synthetic DNA templates.
**FIG. 27** shows a schematic overview of another experiment for adaptor addition by direct hybridization by capture probe and digestion by 3' to 5' exonuclease of template nucleic acid using fragmented genomic DNA.

### DETAILED DESCRIPTION

### I. Overview

The methods disclosed herein can make use of damaged DNA that can have low conversion rate using next generation sequencing (NGS). The methods provided herein can provide higher conversion rate and improved sensitivity due to less DNA loss associated with fewer steps in the process. Furthermore, use of multiple bridge probe- adaptor anchor can increase capture specificity and efficiency. Hence, the methods provided herein can provide a fast easy and flexible design and low-cost solution for target enrichment in NGS.

Disclosed herein, but not part of the invention as defined in the claims, are methods comprising targeted hybridization of a capture probe to a template and proximity attachment of an end of the capture probe to an end of the template, and subsequent amplification of a target sequence in the template utilizing, e.g., attached adaptors. The methods can improve nucleic acid panel design flexibility and practicality compared to a multiplexed-polymerase chain reaction (PCR) next generation sequencing assay and can result in significant time and cost saving. The methods can also preserve high nucleic acid sample complexity and can feature low bias presentation due to a high conversion rate, e.g., using damaged DNA as an input.

Furthermore, localized target capturing methods disclosed herein can eliminate double-strand DNA repair steps and tedious probe hybridization/enrichment. The disclosed methods can be used on single-stranded DNA, damaged DNA, fragmented DNA, e.g., from a formalin fixed paraffin embedded (FFPE) sample, bisulfite treated DNA, and RNA.

### a. Targeted probe addition by direct hybridization overview

Disclosed herein, but not part of the invention as defined in the claims, are methods comprising selectively attaching a capture probe to a template nucleic acid molecule with target sequence by direct hybridization and attachment. A capture probe can be designed to comprise a target specific region that can hybridize to a particular target sequence in a template nucleic acid molecule and thereby can be hybridized to the target template. The proximity brought on by the hybridization can facilitate attachment of the 5' end of the capture probe to the 3' end of the template nucleic acid molecule. The 5' end of the capture probe can be attached to the 3' end of the template by ligation using a ligase. The 5' end of the capture probe can be phosphorylated to facilitate its ligation to the template nucleic acid molecule. The 5' end of the template nucleic acid molecule can be dephosphorylated to reduce self-ligation, e.g., before hybridization of the target specific region of the capture probe to the target sequence in the template.

The capture probe can comprise an adaptor which can act as site of a primer annealing for amplification. The capture probe can further comprise a molecular barcode (MB) to uniquely identify the attached template nucleic acid molecule for later sequencing, detection, identification, measurement, and/or analysis of the template nucleic acid molecule.

### b. Targeted probe addition by indirect association overview

The disclosed methods, which are not part of the invention as defined in the claims, also include certain embodiments comprising selectively attaching an adaptor anchor probe to a template nucleic acid molecule with target sequence by indirect association and attachment. A bridge probe can be designed to hybridize to a particular target sequence in the template nucleic acid molecule and thereby can be hybridized to the target template. An adaptor anchor probe in turn can be designed to hybridize to the bridge probe and can be hybridized to the bridge probe, thereby creating an assembly of three hybridized nucleic acid molecules. The proximity brought on by the hybridization between the three nucleic acid molecules can facilitate the attachment of the 5' end of the adaptor anchor probe to the 3' end of the template nucleic acid molecule. The 5' end of the adaptor anchor probe can be attached to the 3' end of the template by ligation using a ligase. The 5' end of the adaptor anchor probe can be phosphorylated to facilitate its ligation to the template. The 5' end of the template nucleic acid molecule can be dephosphorylated to reduce self-ligation.

The adaptor anchor probe can comprise an adaptor which can act as site of a primer annealing for amplification. The adaptor anchor probe can further comprise a molecular barcode to uniquely identify the attached template for later sequencing, detection, identification, measurement, and/or analysis of the template nucleic acid molecule.

### c. Adaptor addition by direct hybridization overview

Disclosed herein, but not part of the invention as defined in the claims, are methods comprising selectively hybridizing a capture probe to a template nucleic acid molecule with target sequence by direct hybridization. The disclosed methods include certain embodiments comprising adding or incorporating one or more adaptors of the target probe into the extension product without attaching or ligating the target probe to the template nucleic acid. A capture probe can be designed to comprise a target specific region that can hybridize to a particular target sequence in a template nucleic acid molecule and thereby can be hybridized to the target template. The capture probe can further comprise an adaptor which can act as site of a primer annealing for amplification. The adaptor can be positioned at a 5' end of the target specific region.

Upon hybridization with the capture probe, the template nucleic acid molecule can be contacted with 3' to 5' exonuclease to digest any 3' overhang of the template nucleic acid. After digestion, the 3' end of the template nucleic acid molecule can be extended to generate an extension product comprising the sequences complementary of the adaptor.

In some cases, upon hybridization of the capture probe and the template nucleic acid, a 3' end of the capture probe can be extended to generate an extension product comprising the adaptor.

The capture probe can further comprise a molecular barcode (MB) to uniquely identify the attached template nucleic acid molecule for later sequencing, detection, identification, measurement, and/or analysis of the template nucleic acid molecule.

### d. Adaptor addition by indirect association overview

The disclosed invention relates to embodiments comprising selectively hybridizing a first and a second bridge probe to the template nucleic acid. The disclosed methods include certain embodiments comprising adding or incorporating one or more adaptors of the bridge probe into the extension product without attaching or ligating the targeted probe to the template nucleic acid. The bridge probes are designed to hybridize to a particular target sequence in the template nucleic acid molecule and thereby hybridize to the target template. An adaptor anchor probe in turn hybridizes to the bridge probes, thereby creating an assembly of the template nucleic acid molecule, the bridge probes and the adaptor anchor probe. The multiple hybridization assembly can provide more stability to the complex synergistically. The anchor probe in the multiple hybridization assembly is subsequently coupled to a solid support. The first bridge probe further comprises an adaptor sequence which can act as site of a primer annealing for amplification. The adaptor sequence is positioned at a 5' end of the target specific region.

Upon hybridization with the capture probe, the template nucleic acid molecule is contacted with 3' to 5' exonuclease to digest any 3' overhang of the template nucleic acid. After digestion, the 3' end of the template nucleic acid molecule is extended to generate an extension product comprising the sequences complementary of the adaptor sequence. A 3' end of the bridge probe can also be extended to generate an extension product comprising the adaptor sequence upon the hybridization of the bridge probe and the template.

The bridge probes can further comprise a molecular barcode (MB) to uniquely identify the attached template nucleic acid molecule for later sequencing, detection, identification, measurement, and/or analysis of the template nucleic acid molecule.

### II. Probe Addition by Direct Hybridization (not part of the invention as defined in the claims)

Methods disclosed herein can comprise direct hybridization and ligation of the capture probe to a template. The capture probe can comprise an adaptor, to which a primer can be hybridized. Amplification can be performed using the primer for sequencing and/or library construction.

### a. Targeted probe proximity ligation and amplification using adaptor (not part of the invention as defined in the claims)

Disclosed herein is a method comprising hybridizing a target specific region of a capture probe to a target sequence of a template nucleic acid molecule; attaching a 3' end of the template nucleic acid molecule to a 5' end of the capture probe, thereby generating a template nucleic acid molecule attached to the capture probe; hybridizing a first adaptor primer to a first adaptor of the capture probe; extending the first adaptor primer, thereby generating a first extension product; and attaching a second adaptor to a 3' end of the first extension product, thereby generating a first extension product attached to the second adaptor.

The capture probe can comprise a molecular barcode (MB) with unique identifying sequence to uniquely identify the template and/or to assure the specificity of the capture probe to the template nucleic acid molecule. The template nucleic acid molecule can be phosphorylated at its 5' end to facilitate ligation of one strand of an adaptor, e.g., a double-stranded second adaptor, to the template nucleic acid molecule. To amplify the template nucleic acid molecule, a second adaptor primer that hybridizes to the second adaptor can be used along with a first adaptor primer. A primer that hybridizes to target sequence of the template nucleic acid molecule can be used along with the first adaptor primer to amplify the template nucleic acid molecule.

**FIG. 1A** illustrates a method of direct hybridization of a capture probe to a template nucleic acid molecule and ligation of the capture probe to the template. A nucleic acid, e.g., a double stranded (ds) DNA containing a target sequence (hash mark, TS) can be used as template. One of the strands can be damaged (e.g., nicked). The capture probe can comprise of a target specific region (TSR) which is complementary to the TS of the template and a first adaptor (AD1). The 5' end of the capture probe can be phosphorylated. The template can be dephosphorylated at its 5' end. The TSR of the capture probe can be hybridized to the TS of the template and the phosphorylated 5' end of the capture probe can be ligated to 3' end of the template due to the close proximity after hybridization. A first adaptor primer (AD1 primer) can be hybridized to a first adaptor (AD1) of the capture probe and extended, e.g., with a strand displacement mechanism to synthesize a complementary strand as shown in **FIG. 1B. FIG. 1C** illustrates use of the ligated second adaptor (AD2) for further amplification of TS. A double-stranded AD2 can be ligated to the template and its extension product and PCR can be performed using primers that anneal to AD1 and AD2 at each end, thereby amplifying the TS and generating a library. In some cases, a single stranded AD2 adaptor is ligated to a 3' of the extension product. The capture probe can comprise a unique molecular barcode (MB) to uniquely identify the template in subsequent analysis.

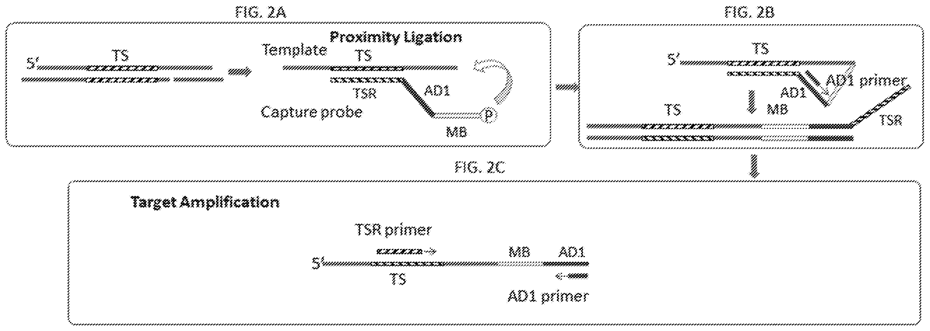

Different primers can be employed for amplification once the capture probe is attached to the template. As shown in **FIG. 2C****,** the target sequence (TS) can be amplified using primers that hybridize to AD1 and the complement of the TS. In another embodiment, the primer can be designed to anneal to regions flanking the complement of the TS (e.g., either 5' or 3' of the complement of the TS).

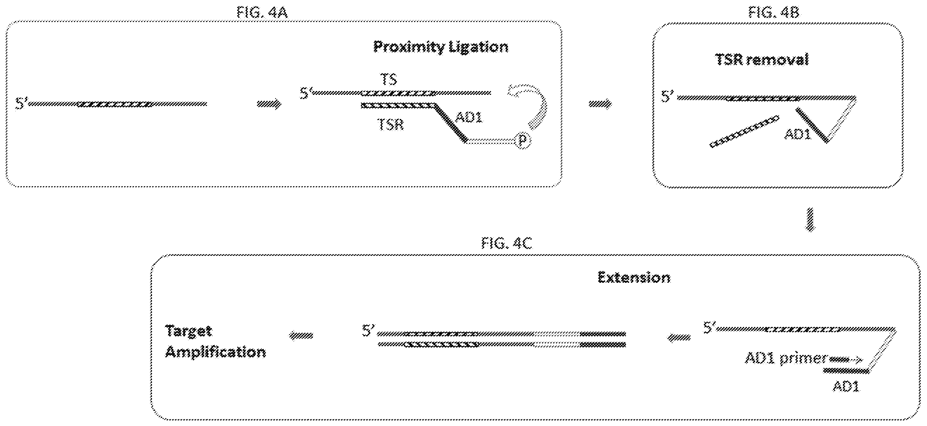

The TSR can be detached or cleaved from the capture probe after ligation of the capture probe to the template. The cleavage can remove a hairpin loop. The original template strand might not be a desirable template for PCR reaction because the hairpin loop formed by the hybridization of TSR and TS and ligation of the capture probe to the template might interfere with primer annealing to AD1 and subsequent extension. The TSR can be cleaved, e.g., between the 5' end of the TSR and 3' end of AD1 by, e.g., restriction enzyme following the proximity ligation of the capture probe to the template, e.g., as shown in **FIGS. 4A** and **4B****.** The cleaved TSR can be removed from the template and AD1 primer can be used in an extension reaction to amplify the template after the TSR is removed (see e.g., **FIG. 4C**).

### b. Targeted probe proximity ligation and amplification using TSR (not part of the invention as defined in the claims)

The 3' end of the capture probe can serve as an extension primer to synthesize a complementary strand to the template nucleic acid molecule without a need for a separate extension primer. **FIGS. 3A-3C** illustrate a possible use of a TSR region of a capture probe to amplify the template after proximity ligation of the capture probe to the template. The hybridized capture probe can form a hairpin loop once its 5' end is ligated to the 3' end of a template (see e.g., **FIG. 3A** and **FIG. 3B****).** The TSR (at the 3' end of the capture probe) can be extended toward the 5' end of the template like a primer, forming complementary sequences that are hybridized to the template (see e.g., **FIGS. 3B-3C****).** A double-stranded AD2 can be ligated to the ends of the looped and extended template (see e.g., **FIG. 3C****).** In some cases, a single stranded AD2 adaptor can be ligated to a 5' end of the template. The 5' end of the template can be phosphorylated to ensure a strand of AD2 ligates to the template. Then the template strand can further be amplified using primers annealing to AD1 and AD2 sequences (see e.g., **FIG. 3C**).

### III. Probe Addition by Indirect Association (not part of the invention as defined in the claims)

A probe can be indirectly associated with a template and proximity ligated to the template. This method can provide flexibility to the design of a capture probe panel and can permit detachment of a TSR from the template after ligation. A bridge probe can comprise a TSR that hybridizes to a TS of the template and adaptor landing sequence (ALS) that hybridizes to bridge binding sequence (BBS) of an adaptor anchor probe. The hybridizations between the template and the bridge probe and between the bridge probe and the adaptor anchor probe can bring the adaptor anchor probe near the template, facilitating the ligation of the adaptor anchor probe to the template. The adaptor anchor probe can further comprise AD1, a unique identifier, MB, and a 5' phosphate.

### a. Targeted probe proximity ligation and amplification using adaptor (not part of the invention as defined in the claims)

Disclosed herein is a method comprising hybridizing a first target specific region (TSR) of a first bridge probe to a first target sequence (TS) of a template nucleic acid molecule, wherein a first adaptor landing sequence (ALS) of the first bridge probe is bound to a first bridge binding sequence (BBS) of an adaptor anchor probe; and attaching (e.g., ligating) a 3' end of the template nucleic acid molecule to a 5' end of the adaptor anchor probe, thereby generating a template nucleic acid molecule attached to the adaptor anchor probe (see e.g., **FIG. 5A** and **FIG. 6A****).** The first bridge probe can further comprise a linker that connects the target specific region (TSR) and ALS (see e.g., **FIG. 5A** and **FIG. 6A**).

The adaptor anchor probe can comprise a MB with unique identifying sequence to uniquely identify the template in subsequent analysis (see e.g., **FIG. 5A** and **FIG. 6A**) and/or to assure the specificity of the capture probe to the template nucleic acid molecule. The template nucleic acid molecule can be phosphorylated at its 5' end to facilitate ligation of one strand of a second adaptor (e.g., a single-stranded or double-stranded adaptor) to the template nucleic acid molecule (see e.g., **FIG. 5A** and **FIG. 6A**). To amplify the template nucleic acid molecule, a second adaptor primer that hybridizes to the second adaptor can be used along with a first adaptor primer. In some cases, a primer that hybridizes to the complement of the target sequence (TS) of the template nucleic acid molecule can be used along with the first adaptor primer to amplify the template nucleic acid molecule (see e.g., **FIG. 6C**). The first target specific region (TSR) of the first bridge probe can be in a 3'-portion of the first bridge probe (see e.g., **FIGS. 5A** and 6A). The first target specific region (TSR) of the first bridge probe can be in a 5'-portion of the first bridge probe (see e.g., **FIG. 7A**). The adaptor anchor probe can be associated with the template through the bridge probe and the phosphorylated 5' end of the adaptor anchor probe can be ligated to the 3' end of the template, thereby attaching the AD1 and MB of the adaptor anchor probe to the template (see e.g., **FIGS. 5A****,** **6A****,** **7A****,** and **8A**). The ligated template can be amplified using either primers that anneal to AD1 and AD2 (see e.g., **FIGS. 5C** and **7C**) or those that anneal to AD1 and the complement of the TS (see e.g., **FIG. 6C**). The adaptor anchor probe can lack a TSR; when the adaptor anchor probe lacks a TSR, it does not form a double strand with the template. In some cases, direct hybridization between the adaptor anchor probe and the template can interfere with primer annealing, e.g., annealing of an AD1 primer to AD1 adaptor in the adaptor anchor probe.

### b. Targeted probe proximity ligation and amplification using TSR (not part of the invention as defined in the claims)

A TSR in a 3' end of the first bridge probe can be extended like a primer to generate a first extension product (see e.g., **FIG. 8B**). A second adaptor (AD2) can be attached to the first extension product and an AD2 primer can be used with AD1 primer to amplify the template (see e.g., **FIG. 8C**). The AD2 can be double stranded and attached to the template and the first extension product, facilitated by phosphorylation of the 5' end of the template (see e.g., **FIG. 8C**). In some cases, AD2 is single-stranded and is ligated to the 5' end of the template. In other embodiments, target sequence can be amplified using primers that anneal to AD1 and the complement of TS.

FIGS. 8A-8C illustrate an example of an extension reaction using TSR (3' end of bridge probe). A TSR at the 3' end of the bridge probe can serve as an extension primer to synthesize a first extension product with sequences complementary to the template (see e.g., **FIG. 8B**). The 5' end of the template can be phosphorylated to facilitate the ligation of a double stranded AD2 (see e.g., **FIG. 8C**). In some cases, a single-stranded AD2 is ligated to the 5' end of the template. The template can be amplified by a primer set annealing to AD1 and AD2 sequences (or the complement of AD2).

### c. Hybridization of multiple targeted probes bridge probes and proximity ligation (not part of the invention as defined in the claims)

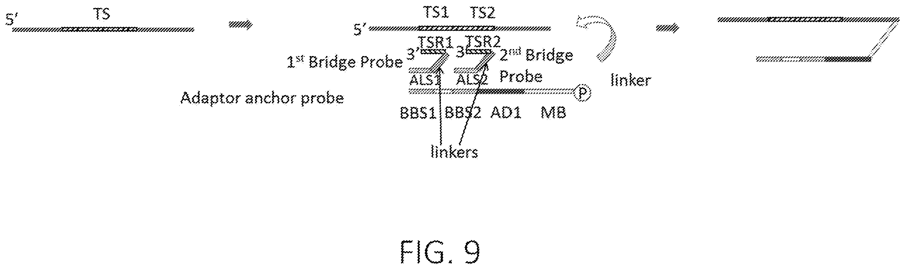

The methods can further comprise hybridizing a second target specific region (TRS2) of a second bridge probe to a second target sequence (TS2) of the template nucleic acid molecule, wherein a second adaptor landing sequence (ALS2) of the second bridge probe is bound to a second bridge binding sequence (BBS2) of the adaptor anchor probe (see e.g., **FIGS. 9-12**). The second bridge probe can further comprise a linker that connects the second target specific region (TSR2) and ALS2 (see e.g., **FIGS. 9-12**). The second bridge probe can comprise a second linker between the second target specific region (TSR2) and the second adaptor landing sequence (ALS2).

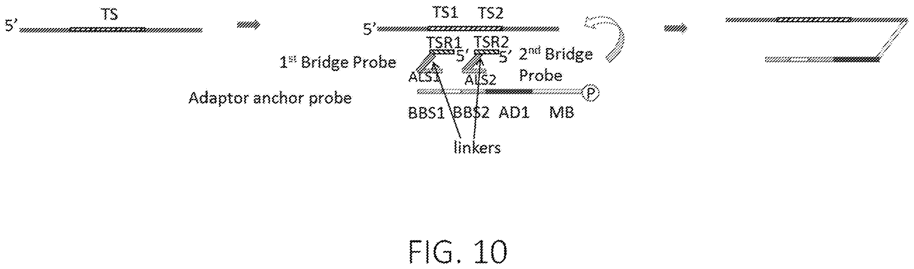

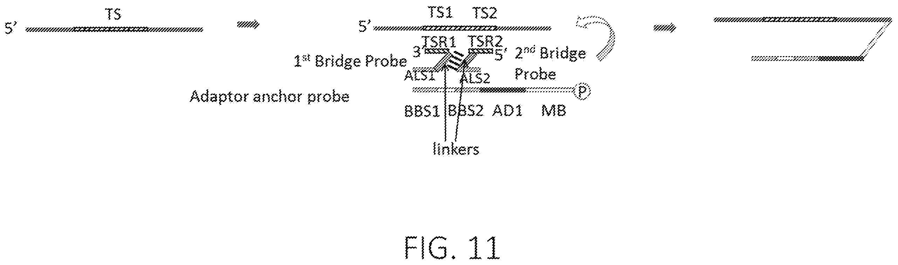

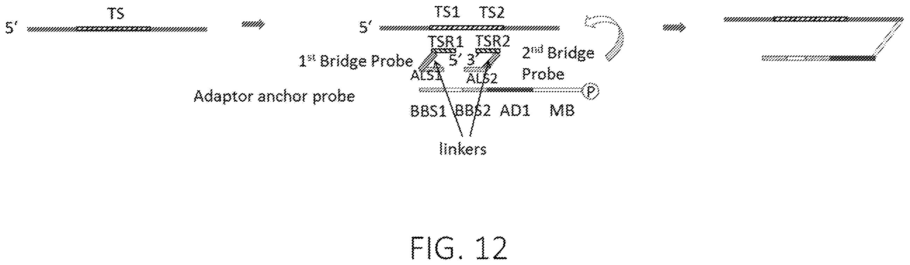

The first target specific region (TSR1) of the first bridge probe can be in a 3'-portion of the first bridge probe and the second target specific region (TSR2) of the second bridge probe can be in a 3'-portion of the second bridge probe, e.g., as illustrated in **FIG. 9****.** The first target specific region (TSR1) of the first bridge probe can be in a 5'-portion of the first bridge probe and the second target specific region (TSR2) of the second bridge probe can be in a 5'-portion of the second bridge probe (see e.g., **FIG. 10**). The first target specific region (TSR1) of the first bridge probe can be in a 5'-portion of the first bridge probe and the second target specific region (TSR2) of the second bridge probe can be in a 3'-portion of the second bridge probe (see e.g., **FIG. 12**). The first target specific region (TSR1) of the first bridge probe can be in a 3'-portion of the first bridge probe and the second target specific region (TSR2) of the second bridge probe can be in a 5'-portion of the second bridge probe (see e.g., **FIG. 11**). A linker of the first bridge probe can further hybridize to a linker of the second bridge probe. The hybridization of the first bridge probe and the second bridge probe can stabilize the four nucleic acid molecule assembly and can facilitate the ligation of the 5' end of the adaptor anchor probe to the 3' end of the template (see e.g., **FIG. 11**).

More than two bridge probes can be used in methods described herein. For example, at least 2, 3, 4, 5, 6, 7, 8, 9, 10, 25, 50, 75, 100, or more bridge probes can be used to bridge a template and an adaptor anchor probe in methods described herein.

### IV. Adaptor Addition by Direct Hybridization (not part of the invention as defined in the claims)

Methods disclosed herein can comprise direct hybridization of the capture probe and template nucleic acid, as well as addition or incorporation of the adaptor of the capture probe into extension product. The capture probe can comprise an adaptor, to which a primer can be hybridized. Amplification can be performed using the primer for sequencing and/or library construction. The capture probe can further comprise molecular barcode. The adaptor addition can also be done without ligating the capture probe to template nucleic acid.

The template nucleic acid molecule can be captured and enriched from low-input samples such as cell-free DNA (cfDNA) and circulating tumor DNA (ctDNA). The capture and enrichment can be done by direct hybridization with capture probe. The capture probe can comprise one or more binding moieties. The binding moiety can be a biotin. The binding moieties can be attached to a support. The support can be a bead. The bead can be a streptavidin bead.

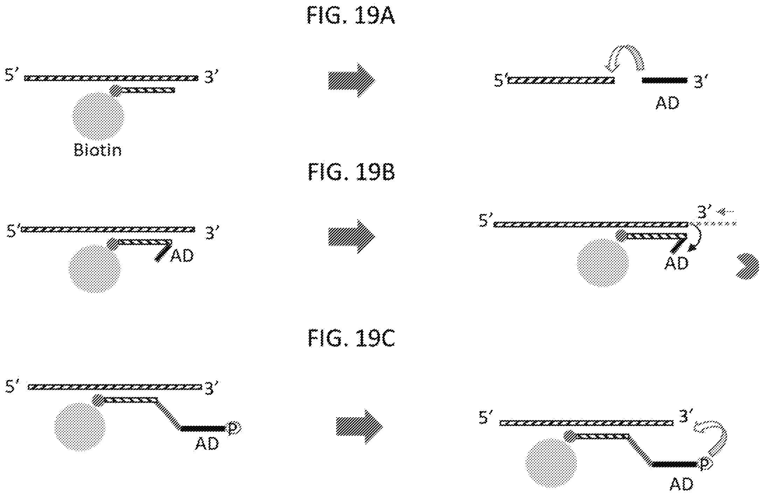

**FIGS. 19A-19C** illustrates various methods of adaptor addition or incorporation after direct hybridization of the capture probe to template nucleic acid. A nucleic acid containing a target sequence (hash mark, TS) can be used as template. The capture probe can comprise of a target specific region (TSR) which is complementary to the TS of the template and an adaptor (AD). **FIG. 19A** shows hybridization or capture of the template nucleic acid with capture probe, followed by direct attachment of the adaptor to the template. **FIG. 19C** shows the attachment of the capture probe comprising an adaptor to the template after hybridization to the template. A nucleic acid containing a target sequence (hash mark, TS) can be used as template. The capture probe can comprise of a target specific region (TSR) which is complementary to the TS of the template and an adaptor (AD).

### a. Adaptor addition after exonuclease digestion (not part of the invention as defined in the claims)

Disclosed herein is a method comprising hybridizing a target specific region of a capture probe to a target sequence of a template nucleic acid molecule, wherein the capture probe further comprises a first adaptor positioned at a 5' end of the target specific region; contacting the template nucleic acid molecule with 3' to 5' exonuclease after hybridizing the target specific region; extending a 3' end of the template nucleic acid molecule, thereby generating a first extension product. The method further comprises hybridizing a primer comprising the first adaptor to the first extension product; and extending the primer comprising the first adaptor, thereby generating a second extension product. In some cases, a 3' end of the capture probe can be extended to generate a second extension product.

FIG. 19B illustrates the hybridization or capture of the template nucleic acid molecule with capture probe, followed by the digestion of the single-stranded 3' overhang with 3' to 5' exonuclease. After the digestion, the template nucleic acid is extended using the adaptor of capture probe as a template, thus incorporating the adaptor into the extension product.

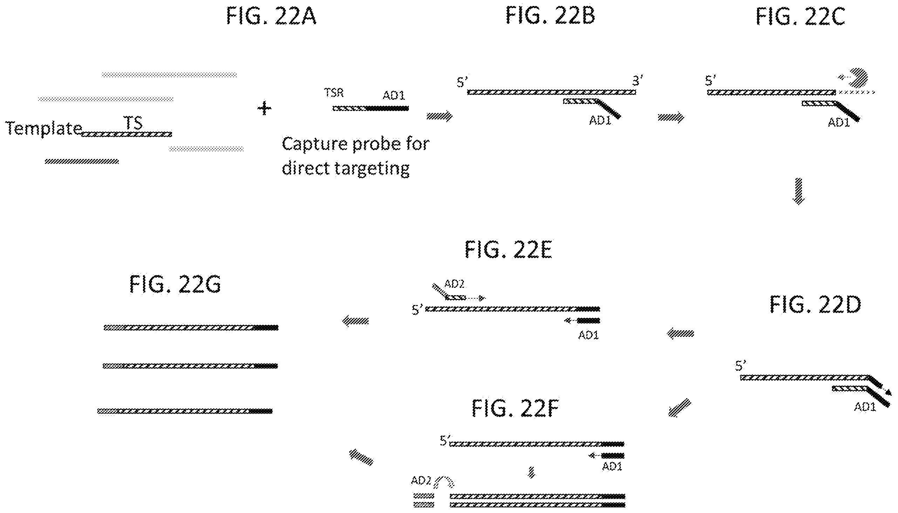

**FIGS. 22A-22G** further illustrate the steps for hybridization, digestion, and extension. **FIG. 22E** shows the hybridizing a target specific primer to the second extension product, wherein the target specific primer is attached to a second adaptor; and extending the target specific primer to generate a third extension product. The second extension product and the third extension product can be further amplified to generate amplified products of **FIG. 22G****.**

In some cases, the disclosed method can comprise attaching a second adaptor to a 5' end of the first extension product as shown in **FIG. 22F****.** The 5' end of the first extension product can be phosphorylated to facilitate attachment the second adaptor. The second adaptor can be a double-stranded adaptor, wherein one of two strands of the second adaptor attaches to the second extension product. After the attachment of the second adaptor, the first extension product and the second extension product can be amplified to generate the amplified products of **FIG. 22G****.**

### b. Adaptor addition after capture probe extension (not part of the invention as defined in the claims)

The hybridized or captured template nucleic acid molecule can also be amplified by extending a 3' end of the capture probe to generate a first extension product, without a need for a separate extension primer. The capture probe can comprise an adaptor.

The methods can further comprise attaching a second adaptor to a 3' end of the first extension product, thereby generating a first extension product attached to the second adaptor. The 5' end of the template nucleic acid can be phosphorylated to facilitate the attachment or ligation of the second adaptor to the template. The second adaptor can be a double-stranded adaptor. One of the two strands of the double-stranded second adaptor can be attached to the template nucleic acid molecule. A second adaptor primer can be hybridized to the second adaptor attached to the first extension product and extended to generate a second extension product. The first extension product attached to the second adaptor and the second extension product can be amplified to generate amplified products.

In some cases, a target specific primer can be hybridized to the first extension product that is attached to a second adaptor. The target specific primer can be extended to synthesize a complementary strand (a second extension product) to the first extension product that is attached to the second adaptor. The first extension product and the second extension product can be amplified to form more amplified products.

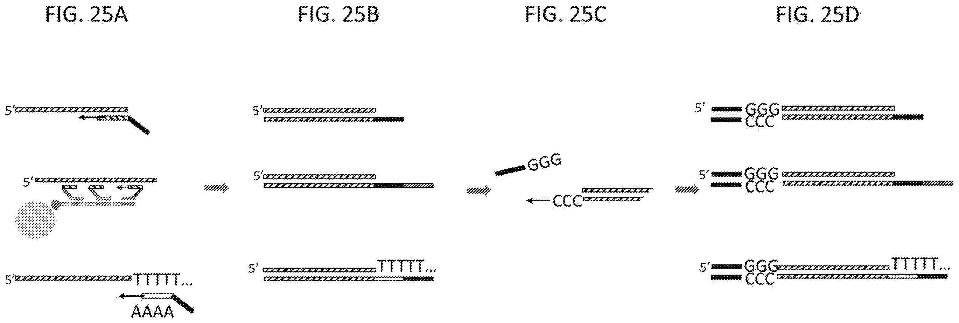

Adaptors can be added by to the extension product by extending the hybridized target probe attached to the adaptor through either direct indirect interaction (see **FIG. 25A****,** top and middle). Thymine can be added to the DNA template by terminal nuclease transferase (see **FIG. 25A****,** bottom). A poly-adenine (polyA) oligonucleotide can be hybridized to the 3'-polyT of the extended template. As illustrated in **FIG. 25B****,** the polyA oligonucleotide can be attached to adaptor such that when 3' end of the polyA is extended, the first extension product can comprise the first adaptor. A poly-cytosine (polyC) can be added to the 3' end of the extension product, which may complementarily bind with poly-guanine (polyG) primer attached to a second adaptor (see **FIG. 25C****).** The polyG-adaptor can then serves as a template to allow for the nucleotide addition process to continue and the second adaptor added to the first extension product (see **FIG. 25D****).**

### V. Adaptor Addition by Indirect Association

According to the present invention as defined in the claims, the target probe hybridization can be facilitated by synergistic interaction of template nucleic acid, a first bridge probe, a second bridge probe and an anchor probe that form an assembly. The multi-complex assembly can stabilize the hybridization interaction between the template and the target probes. The first bridge probe and second bridge probe comprise a target specific region that hybridizes to a target region of the template and adaptor landing sequence (ALS) that hybridizes to bridge binding sequence (BBS) of an adaptor anchor probe. The hybridizations between the template and the bridge probes and between the bridge probes and the adaptor anchor probe forms a multi-complex assembly. The first bridge probe further comprises an adaptor sequence positioned 5' of the target specific region, and can further comprise a unique identifier, MB, and/or a 5' phosphate. According to the present invention, after formation of the multi-complex assembly, the anchor probe is coupled to a solid support and the complex is subsequently contacted with a 3' to 5' exonuclease, wherein the 3' to 5' exonuclease cleaves the 3' end of the template nucleic acid molecule. A first extension product is synthesized by using the adaptor sequence of the first bridge probe as a template. More than two bridges probes can be used in the methods disclosed herein. For example, at least 2, 3, 4, 5, 6, 7, 8, 9, 10, 25, 50, 75, 100, or more bridge probes can be used to bridge the template and the adaptor anchor probe.

The template nucleic acid can be captured and enriched from low-input samples such as cell-free DNA (cfDNA) and circulating tumor DNA (ctDNA). The capture and enrichment is done by the indirect association with adaptor anchor probe through hybridization with a first and a second bridge probe. The bridge probes and/or adaptor anchor probe can comprise one or more binding moieties. The binding moiety can be a biotin. The binding moieties can be attached to a support. The support can be a bead. The bead can be a streptavidin bead.

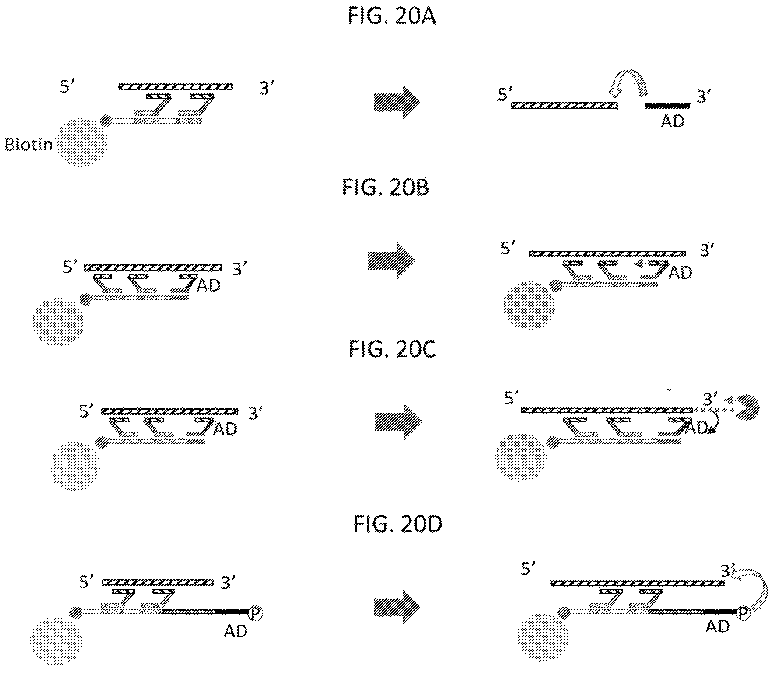

**FIGS. 20A-20D** show different methods of adding or incorporating an adaptor to the template nucleic acid, following hybridization or capture with bridge probe and adaptor anchor probe. **FIG. 20A** illustrates the ligation of the adaptor to the captured template nucleic acid. **FIG. 20D** shows the proximity ligation of the adaptor anchor probe comprising an adaptor to the template after the hybridization.

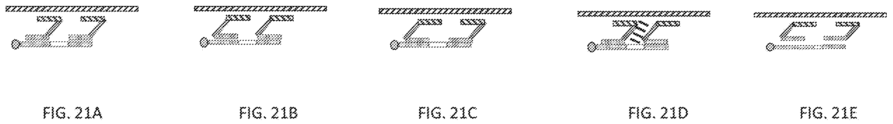

The methods of the present invention comprise hybridizing a second target specific region of a second bridge probe to a second target sequence of the template nucleic acid molecule, wherein a second adaptor landing sequence of the second bridge probe binds to a second bridge binding sequence of the adaptor anchor probe (see e.g., **FIGS. 21A-21E****).** The second bridge probe can further comprise a linker that connects the second target specific region and the second adaptor landing sequence.

### a. Adaptor addition after bridge probe extension (not part of the invention as defined in the claims)

Disclosed herein, but not part of the invention as defined in the claims, is a method comprising hybridizing a first target specific region of a first bridge probe to a first target sequence of a template nucleic acid molecule, wherein a first adaptor landing sequence of the first bridge probe can be bound to a first bridge binding sequence of an adaptor anchor probe; hybridizing a second target specific region of a second bridge probe to a second target sequence of the template nucleic acid molecule, wherein a second adaptor landing sequence of the second bridge probe can be bound to a second bridge binding sequence of the adaptor anchor probe; and extending a 3' end of the first bridge probe, thereby generating a first extension product. The first bridge probe can comprise a first adaptor.

The method can further comprise attaching a second adaptor to a 3' end of the first extension product. The second adaptor can be a double-stranded adaptor. One of the two strands of the second adaptor can attach to the template nucleic acid molecule. Prior to attaching the second adaptor to the template, the 5' end of the template nucleic acid molecule can be phosphorylated.

Alternatively, a target specific primer can be hybridized to the first extension product and can be extended to generate a second extension product. The target specific primer can be attached to a second adaptor. A primer comprising the first adaptor can be hybridized to the second extension product and can be extended to generate a third extension product. Amplifying the second extension product and third extension product can generate further amplified products.

**FIG. 20B** shows the incorporation of adaptor into the extension product by extending the 3' end of the bridge probe using the target nucleic acid molecule as the template.

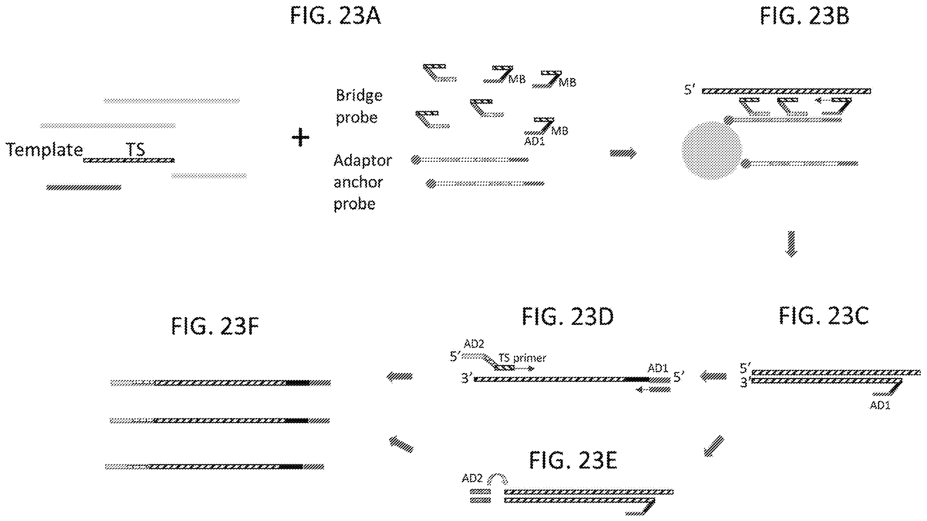

**FIGS. 23A-23F** illustrate in the incorporation of one or more adaptors in the extension products. **FIGS. 23A- 23C** show the hybridization and extension of the first bridge probe, as well as the first extension product. A target specific primer can be hybridized to the first extension product and extended to synthesize a complementary strand to the first extension product, and a primer comprising the first adaptor can be hybridized to the second extension product generate sequences complementary to the second extension product (see **FIG. 23D**). The second extension product and the third extension product can be amplified to generate the amplified products shown in **FIG. 23F****.** In another case, a second (double-stranded) adaptor can be attached to a 3' end of the first extension product and a 5' end of the template nucleic acid molecule (see **FIG. 23E**). The 5' end of the template nucleic acid molecule can be phosphorylated prior to the attachment of the second adaptor. The bridge probe may further comprise molecular barcodes which can be incorporated in the extension products to uniquely identify the attached template for later sequencing, detection, identification, measurement, and/or analysis of the template nucleic acid molecule.

Adaptors can be added or incorporated to the extension product by extending the hybridized bridge probe attached to the adaptor. The adaptor can also be added to the extension product by first performing 3' primer extension to the template nucleic acid molecule. Polythymine (polyT) oligonucleotides can be added to a 3' end of the DNA template by a terminal nucleic transferase. A poly-adenine (polyA) oligonucleotide can be hybridized to the 3'-polyT of the extended template. The polyA oligonucleotide can be attached to adaptor such that when 3' end of the polyA is extended, the first extension product can comprise the first adaptor. A poly-cytosine (polyC) can be added to the 3' end of the first extension product, which may complementarily bind with poly-guanine (polyG) primer attached to a second adaptor. The polyG-adaptor can serve as a template, the first extension product can further extend to add the second adaptor.

### b. Adaptor addition after exonuclease digestion

Upon hybridizing with the bridge probe, the template may have single-stranded 3' overhang. The 3' overhang is digested with enzymes acting as 3' to 5' exonuclease. The present invention provides a method comprising hybridizing a first target specific region of a first bridge probe to a first target sequence of a template nucleic acid molecule, wherein a first adaptor landing sequence of the first bridge probe is bound to a first bridge binding sequence of an adaptor anchor probe, wherein the first bridge probe further comprises a first adaptor sequence positioned at a 5' end of the target specific region; hybridizing a second target specific region of a second bridge probe to a second target sequence of the template nucleic acid molecule, and hybridizing a second landing sequence of the second bridge probe to a second bridge binding sequence of the anchor probe, thereby forming a complex comprising the template nucleic acid molecule, the first bridge probe, the second bridge probe, and the anchor probe; subsequent to the formation of the multi-complex assembly, coupling the anchor probe to the solid support and contacting the template nucleic acid molecule with 3' to 5' exonuclease after hybridizing the target specific region; and extending a 3' end of the template nucleic acid molecule using the first adaptor as a template, thereby generating a first extension product (see **FIG. 20C****).**

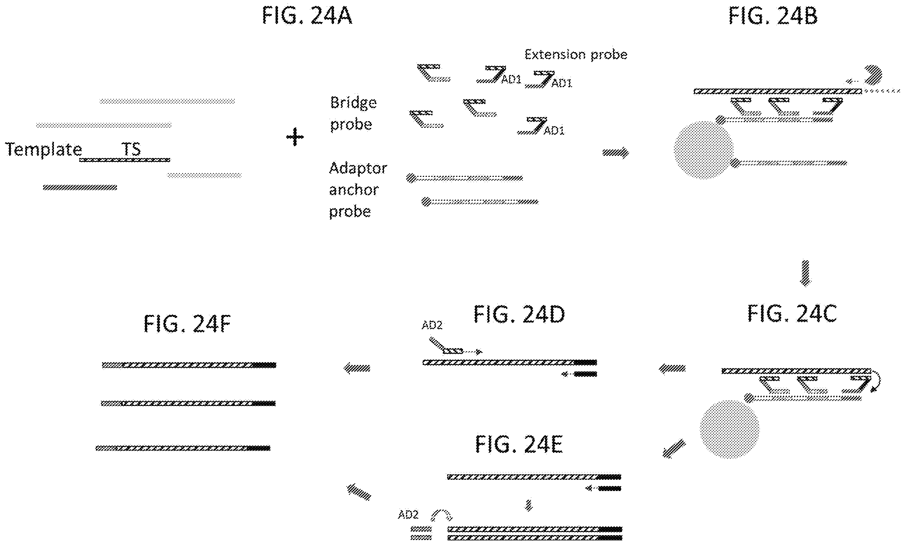

**FIGS. 24A-24F** further illustrate the steps of incorporating one or more adaptors in the extension products after digestion with 3' to 5' exonuclease. **FIGS. 24A-24C** show the hybridization of multiple bridge probes to the template and the adaptor anchor probe, followed by extension of a 3' end of the template using a first adaptor of the first bridge probe as template to generate a first extension product comprising sequences complementary to the first adaptor. The first extension product can be hybridized with a primer comprising the first adaptor and the 3' end of the primer can be extended to generate a second extension product (see **FIGS. 24D-****24E).** A target specific primer can be hybridized to the second extension product and the 3' end can be extended to generate a third extension product (see **FIG. 24D****).** A second adaptor can be attached to the target specific primer prior to the hybridization of the target specific primer to the first extension product. The second extension product and the third extension product can be amplified to generate more amplified products (see **FIG. 24F****).** As illustrated in **FIG. 24E****,** a second (double-stranded) adaptor can be attached to the 5' end of the first extension product and the 3' end of the second extension product. The first extension product may need to be phosphorylated prior to the attachment to the second adaptor.

In some cases, the 3' end of the template nucleic acid molecule can be extended before, simultaneously, or after the extension of the 3' end of the first bridge probe to generate the first extension product and the second extension product. The template nucleic acid molecule is contacted with the 3' to 5' exonuclease prior to extension of the 3' of the template nucleic acid to remove any 3' overhang.

### VI. Workflows for bisulfite conversion

Provided herein are methods for bisulfite treatment of nucleic acids. The bisulfite treated nucleic acids can be used to study methylation of the nucleic acids. The bisulfite treatment can convert unmethylated cytosines to uracils. Methylation of a cytosine (e.g., 5'-methylctyosine) can prevent bisulfite from converting methylated cytosine to uracil.

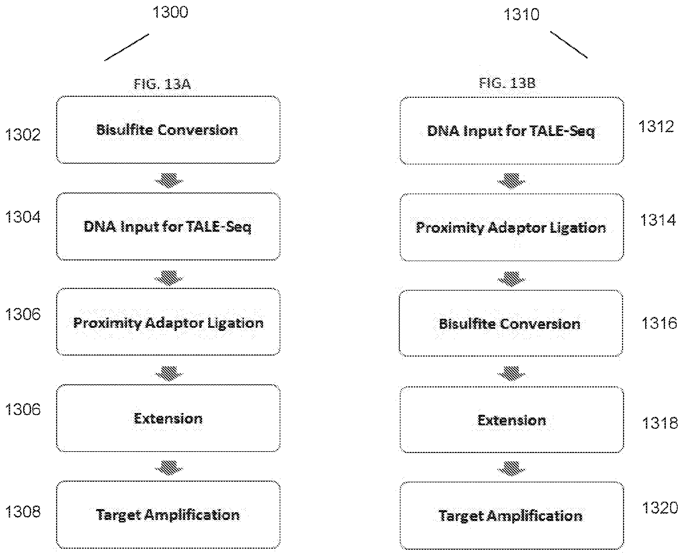

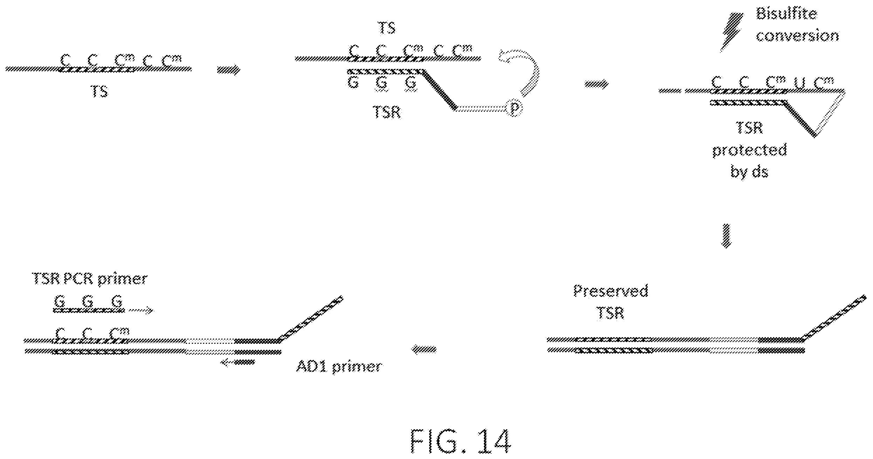

After ligation of a capture probe to a template, the resulting product can be treated with bisulfite (see e.g., FIG. 13B). Formation of double strand sequence (e.g., between a TS of template and TSR of a capture probe) can protect against conversion of cytosines in the hybridized region to uracils during bisulfite treatment (see, e.g., FIG. 14). The double stranded sequence formed by the hybridization of a bridge probe to a template and to an adaptor anchor probe can provide protection against bisulfite conversion of cytosines in the hybridized regions to uracils. Furthermore, since bisulfite treatment can convert non-methylated cytosine to uracil, the protection against conversion of cytosines to uracils at the TS area can allow for the use of amplification primers designed to anneal to the non-bisulfite converted DNA. For the pre-bisulfite conversion capture, the probe can also be designed against the unconverted sequence. Probes and primers that anneal to unconverted cytosines can be more straightforward to design and provide better hybridization.

### a. Bisulfite treatment after hybridization and/or ligation of a target probe to a template nucleic acid

A template nucleic acid (e.g., DNA) can be used for Targeted Adaptor proximity Ligation and Enrichment (TALE) and subsequent sequencing (TALE-SEQ) as described herein (see e.g., **FIG. 13B** (**1310**); (**1312**)). The template nucleic acid (e.g., DNA) can be, e.g., genomic DNA, or cfDNA. A template nucleic acid (e.g., DNA) can be attached to a capture probe or adaptor anchor probe by proximity ligation, e.g., as described herein, e.g., as illustrated in **FIG. 1A** **-** **FIG. 12** and **FIG. 17** **(1314).** The template nucleic acid (e.g., DNA) attached to the capture probe or adaptor anchor probe can be treated with bisulfite **(1316),** extended **(1318),** and amplified subsequently **(1320),** e.g., for methylation sequencing. In some cases, the bisulfite treatment can occur before detachment of a TSR of the capture probe (see e.g., **FIG. 4B****).** In some cases, the bisulfite treatment can be performed after hybridization of the capture probe or bridge probe to the template nucleic acid molecule. The template nucleic acid molecule hybridized to the capture probe or the bridge probe can be treated with bisulfite, extended, and amplified subsequently for methylation sequencing.

The target specific region of a capture probe or a bridge probe can be designed based on the target sequence of the template nucleic acid molecule, and the target sequence of the template nucleic acid molecule can retain non-methylated cytosine after the bisulfite treatment.

**FIG. 14** illustrates the protection of unmethylated cytosines in the TS and TSR sites from conversion to uracil during bisulfite treatment that occurs after hybridization of the TS and TSR and ligation of the capture probe to the template. In this example, a capture probe is hybridized to a template and a 5' end of the capture probe is proximity ligated to the 3' end of the template, forming a double strand region and hairpin loop (see e.g., **FIG. 14****).** Subsequently, the attached template is treated with bisulfite during which the non-methylated cytosines in the hybridized TSR-TS region are not converted to uracil, whereas a non-methylated cytosine in the single stranded area (in the loop) is converted to uracil. The protection against conversion of cytosines to uracils at the TS area can allow for the use of probes designed to anneal to the non-bisulfite converted DNA. In some cases, one or more cytosine residues in a primer binding site (e.g., an adaptor and/or in a template) are not protected from bisulfite conversion. Following bisulfite conversion, a primer binding site in an adaptor can comprise one or more uracils. A primer can be designed to be complementary to the adaptor sequence comprising one or more uracils. The primer can be 100% complementary to the adaptor sequence comprising one or more uracils, or less than 100% complementary to the adaptor sequence comprising one or more uracils.

A template can comprise one or more uracils after bisulfite treatment. A primer annealing to an adaptor can use the template comprising the one or more uracils for strand extension. The extended strand can comprise one or more adenines that are base-paired to the one or more uracils. The extension product can be denatured from the template. A primer can be annealed to the extension product in the region comprising the one or more adenines and extended. The primer can be used in amplification of the template with, e.g., an adaptor primer.

### b. Bisulfite treatment before hybridization and/or ligation of a target probe to a template

Template nucleic acid molecules can be bisulfite treated prior to hybridization to capture probes or bridge probes and ligation to capture probes or adaptor anchor probes (see e.g., **FIG. 13A** **(1300)).** DNA can be treated with bisulfite to convert unmethylated cytosines to uracils

**(1302).** The bisulfite treated DNA can be used as an input for Targeted Adaptor proximity Ligation and Enrichment **(TALE)** and subsequent sequencing **(TALE-SEQ) (1304).** The bisulfite treated DNA can be used as input for proximity adaptor ligation **(1306),** e.g., as illustrated in **FIG. 1A** - **FIG. 12** and **FIG. 17****.** The TSR of a probe can be designed to anneal to the template in which existing non-methylated cytosines have been converted to uracil. Following proximity adaptor ligation, extension **(1306)** can be performed followed by target amplification **(1308).**

### VII. Clean-up/specificity

### a. Clean-up of off-target nucleic acid molecule or random ligation products using exonuclease

In some cases, a 5' end of a capture probe can ligate to a 3' end of a template without a TSR of the capture probe annealing to the template. A 5' end of an adaptor anchor probe can ligate to a 3' end of a template without the adaptor anchor probe associating with the template through a bridge probe. The products of these ligation events can be unwanted. Exonuclease can be added to the resulting nucleic acid molecule mix after the direct hybridization and ligation steps to digest any nonspecific attachment of a probe to a template.

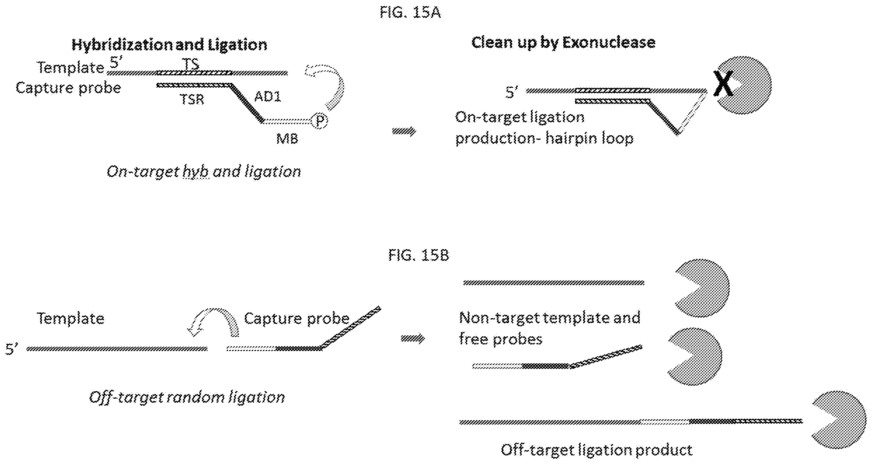

In an example, a 5' end of a capture probe can ligate to a 3' end of a template without the TSR of the capture probe annealing to the TS of the template, resulting in a linear molecule (e.g., a molecule lacking a stem loop) (see e.g., **FIG. 15B****).** Any specific attachment, owning to hybridization of a TSR of a capture probe with a TS of a template, can result in a hairpin loop that can protect the template from being digested with exonuclease (e.g., a 3' to 5' exonuclease) (see e.g., **FIG. 15A). FIG. 15A** shows a specific hybridization of a capture TSR to a template TS and ligation of the 5' end of the capture probe to the 3' end of the template. The hybridization and ligation can form a hairpin loop that protects it from exonuclease (e.g., a 3' to 5' exonuclease) digestion. The 5' end of the template can comprise a block that can prevent cleavage by a 5' to 3' exonuclease. **FIG. 15B** shows ligation of a 5' end of a capture probe to a 3' end of template, wherein the TSR of the template does not anneal to the TS of the template, of the template does not comprise a TS. In cases in which one or more bridge probes are used, a 5' end of an adaptor anchor probe can ligate to a 3' end of a template with the adaptor anchor probe associating with the template through the one or more bridge probes. The ligation does not result in hairpin loop formation to protect against exonuclease (e.g., 3' to 5' exonuclease) digestion, leading to degradation of the off-target random ligation product. FIG 15B illustrates an exonuclease (e.g., a 3' to 5' exonuclease) cleaving a free template, a free capture probe, and an off-target ligation product of a capture probe and a template. Exonuclease cleavage of the off-target ligation products can result in selection of ligation products in which a capture probe specifically hybridized to a template and the capture probe attached (e.g., ligated) to the template. Following exonuclease treatment, a purification step can be performed to remove the exonuclease from the ligation products before subsequent extension and amplification steps are performed.

### b. Solid phase extraction

Methods are provided herein to select for templates that are hybridized to a bridge probe (or templates associated with an adaptor anchor probe via a bridge probe), e.g., before the adaptor anchor probe is ligated to the template. The methods can employ solid phase extraction. The methods can be used to increase the likelihood that an adaptor anchor probe ligates to a template specifically associated with the adaptor anchor probe. Methods are provided herein to bind a capture probe, bridge probe, or adaptor anchor probe to a solid support. Suboptimal specificity can be introduced by the possibility that the adaptor anchor probe attaches (e.g., ligates) to the template independent of bridge probe. To reduce such non-specific ligation products as well as unbound probe, labels (e.g., biotin) and capture moieties (e.g., streptavidin beads) can be utilized.

The capture probe, bridge probe, or adaptor anchor probe can comprise a label. The disclosed methods can further comprise capturing to capture probe, the bridge probe, or the adaptor anchor probe by the label. The label can be biotin. The label can be a nucleic acid sequence, such as poly A or Poly T, or specific sequence. The nucleic acid sequence can be about 5 to 30 bases in length. The nucleic acid sequence can comprise DNA and/or RNA. The label can be at the 3' end of the capture probe, bridge probe, or adaptor anchor probe. The label can be a peptide, or modified nucleic acid that can be recognized by antibody such as 5-Bromouridine, and biotin. The label can be conjugated to the capture probe, bridge probe, or adaptor anchor probe by reactions such as "click" chemistry. "Click" chemistry can allow for the conjugation of a reporter molecule like fluorescent dye to a biomolecule like DNA. Click Chemistry can be a reaction between and azide and alkyne that can yield a covalent product (e.g., 1,5-disubstituted 1,2,3-triazole). Copper can serve as a catalyst.

The label can be captured on a solid support. The solid support can be magnetic. The solid support can comprise a bead, flow cell, glass, plate, device comprising one or more microfluidic channels, or a column, The solid support can be a magnetic bead.

The solid support (e.g., bead) can comprise (e.g., by coated with) one or more capture moieties that can bind the label. The capture moiety can be streptavidin, and the streptavidin can bind biotin. The capture moiety can be an antibody. The antibody can bind the label. The capture moiety can be a nucleic acid, e.g., a nucleic acid comprising DNA and/or RNA. The nucleic acid capture moiety can bind a sequence on, e.g., an adaptor anchor probe or bridge probe. In some cases, an anti-RNA/DNA hybrid antibody bound to a solid surface can be used as a capture moiety.

The label and the capture moiety can bind through one or more covalent or non-covalent bonds. Following capture of the capture probe, bridge probe, or adaptor anchor probe on the solid support, the solid support can be washed to remove, e.g., unbound template from the sample. In some cases, no wash step is performed. The wash can be stringent or gentle. The captured capture probe, bridge probe, or adaptor anchor probe (and associated template) can be eluted, e.g., by adding free biotin to the sample when the label is biotin and the capture moiety is streptavidin.

Ligation of the 5' end of the capture probe to the 3' end of the template can occur while the capture probe is captured on the solid support. Ligation of the 5' end of the capture probe to the 3' end of the template can occur after a capture probe/template complex is eluted from a solid support. The 5' end of the capture probe or adaptor anchor probe can be phosphorylated while the capture probe or adaptor anchor probe is captured on a solid support.

Ligation of the 5' end of the adaptor anchor probe to the 3' end of the template can occur while the adaptor anchor probe (and associated bridge probe(s) and template) is captured on the solid support. Ligation of the 5' end of the adaptor anchor probe to the 3' end of the template can occur after the adaptor anchor probe (and associated bridge probe(s) and template) is eluted from the solid support. The 5' end of the capture probe or adaptor anchor probe can be phosphorylated after elution from a solid support.

Extension steps (e.g., extension of an AD1 primer that anneals to an AD1 adaptor in a capture probe) can be performed while the capture probe or adaptor anchor probe are captured on a solid support or after elution of the capture probe (and ligated template) or adaptor anchor probe (and ligated template) are eluted from the solid support.

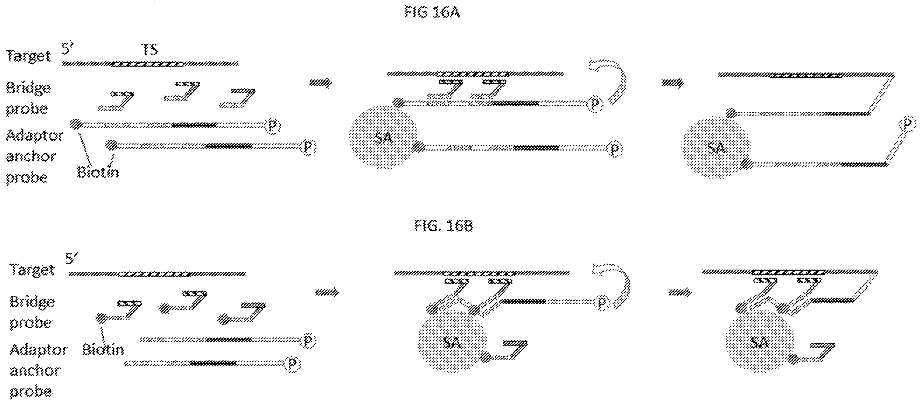

**FIG. 16A** illustrates performing cleanup using streptavidin beads after template, bridge probe, and adaptor anchor probe hybridization, wherein the 3' end of the adaptor anchor probe is biotinylated. Both the hybridization complex and the free adaptor anchor adaptor can bind to the bead. The unbound template and bridge probe can be washed away. Proximity ligation can still happen with the hybridization complex. Ligation can be performed while the complex is on the bead. Ligation can be performed after eluting the complex from the bead. However, for a free adaptor anchor probe the possibility to ligate to any template DNA can be dramatically reduced. **In** **FIG. 16B****,** the 5' end or the 3' end of a first and or second bridge probe can be biotinylated. Streptavidin beads can be used to remove the unhybridized adaptor anchor adaptor and template, which can prevent random ligation of an adaptor anchor probe and a template.

### VIII. Template nucleic acid molecules

The template nucleic acid can be DNA or RNA. The DNA can be genomic DNA (gDNA), mitochondrial DNA, viral DNA, cDNA, cfDNA, or synthetic DNA. The DNA can be double-stranded DNA, single-stranded DNA, fragmented DNA, or damaged DNA. RNA can be mRNA, tRNA, rRNA, microRNA, snRNA, piRNA, small non-coding RNA, polysomal RNA, intron RNA, pre-mRNA, viral RNA, or cell-free RNA.

The template nucleic acid can be naturally occurring or synthetic. The template nucleic acid can have modified heterocyclic bases. The modification can be methylated purines or pyrimidines, acylated purines or pyrimidines, alkylated riboses, or other heterocycles. The template nucleic acid can have modified sugar moieties. The modified sugar moieties can include peptide nucleic acid. The template nucleic acid can comprise peptide nucleic acid. The template nucleic acid can comprise threose nucleic acid. The template nucleic acid can comprise locked nucleic acid. The template nucleic acid can comprise hexitol nucleic acid. The template nucleic acid can be flexible nucleic acid. The template nucleic acid can comprise glycerol nucleic acid.

The template nucleic acid molecule can be captured and enriched from low-input (e.g. 1 ng of nucleic acid materials) samples such as cell-free DNA (cfDNA) and circulating tumor DNA (ctDNA). The low-input samples can have 1 ng, 2 ng, 3 ng, 4 ng, 5 ng, 6 ng, 7 ng, 8 ng, 9 ng, 10 ng, or more of nucleic acid materials. The low-input samples can have less than 10 ng, 9 ng, 8 ng, 7 ng, 6 ng, 5 ng, 4 ng, 3 ng, 2 ng, 1 ng, or less of nucleic acid materials. The low-input samples can have from 200 pg to 10 ng of nucleic acid materials. The low-input samples can have less than 10 ng of nucleic acid materials. The low-input sample can less than 10 ng, 5 ng, 1 ng, 100 pg, 50 pg, 25 pg, or less of the nucleic acid materials. In some cases, the input samples can have 1 ng, 10 ng, 20 ng, 30 ng, 40 ng, 50 ng, or more of nucleic acid molecule. The input samples can have less than 50 ng, 40 ng, 30 ng, 20 ng, 10 ng, 1 ng, or less of nucleic acid materials. The capture and enrichment can be done by target probe hybridization. The target probe can be capture probe, bridge probe, and/or adaptor anchor probe. The target probe can comprise one or more binding moieties. The binding moiety can be a biotin. The binding moieties can be attached to a support. The support can be a bead. The bead can be a streptavidin bead.

The template nucleic acid can be damaged. The damaged nucleic acid can comprise altered or missing bases, and/or modified backbone. The template nucleic acid can be damaged by oxidation, radiation, or random mutation. The template nucleic acid can be damaged by bisulfite treatment.

For damaged DNA, the present disclosure can eliminate double-strand DNA repair steps, providing higher conversion rate and improved sensitivity due to less DNA loss from fewer steps in the process.

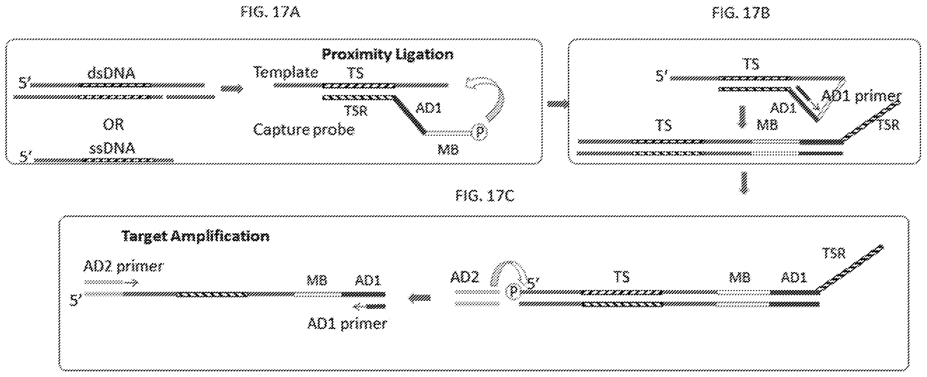

**FIG. 17A** illustrates the use of either damaged dsDNA (with a nick) or ssDNA as template for a library construction. The damage can be due to bisulfite treatment. For the damaged dsDNA, the dsDNA can be denatured so at least one undamaged strand can be used as a template. The template can then be hybridized and attached to a capture probe and amplified using various primers.

The template can be derived from cell-free DNA (cfDNA) or circulating tumor DNA (ctDNA). The cfDNA can be fetal or tumor in source. The template can be derived from liquid biopsy, solid biopsy, or fixed tissue of a subject. The template can be cDNA and can be generated by reverse transcription. The template nucleic acid can be derived from fluid samples, including not limited to plasma, serum, sputum, saliva, urine, or sweat. The fluid samples can be bisulfite treated to study the methylation pattern of the template nucleic acid and/or to determine the tissue origin of the template nucleic acid. The template nucleic acid can be derived from liver, esophagus, kidney, heart, lung, spleen, bladder, colon, or brain. The template nucleic acid can be treated with bisulfite to analyze methylation pattern of organ the template nucleic acid is derived from. The subject can suffer from methylation related diseases such as autoimmune disease, cardiovascular diseases, atherosclerosis, nervous disorders, and cancer.

The template nucleic acid can be derived from male or female subject. The subject can be an infant. The subject can be a teenager. The subject can be a young adult. The subject can be an elderly person.

The template nucleic acid can originate from human, rat, mouse, other animal, or specific plants, bacteria, algae, viruses, and the like. The template nucleic acid can originate from primates. The primates can be chimpanzees or gorillas. The other animal can be a rhesus macaque. The template also can be from a mixture of genomes of different species including host-pathogen, bacterial populations, etc. The template can be cDNA made from RNA expressed from genomes of two or more species.

The template nucleic acid can comprise a target sequence. The target sequence is an exon. The target sequence is can be an intron. The target sequence can comprise a promoter. The target sequence can be previously known. The target sequence can be partially known previously. The target sequence can be previously unknown. The target sequence can comprise a chromosome, chromosome arm, or a gene. The gene can be gene associated with a condition, e.g., cancer.

The template nucleic acid molecule can be dephosphorylated before hybridization to, e.g, reduce the rate of self-ligation.

### IX. Capture probes

Capture probe can be used to capture a template nucleic acid molecule with target sequence. The capture probe can comprise DNA. The capture probe can comprise of RNA. The capture probe can comprise of uracil or methylated cytosine. In some cases, the capture probe does not comprise uracil.

The capture probe can comprise about 400 nucleotides. The capture probe can comprise about 300 nucleotides. The capture probe can comprise about 200 nucleotides. The capture probe can comprise about 180 nucleotides. The capture probe can comprise about 150 nucleotides. The capture probe can comprise about 120 nucleotides. The capture probe can comprise about 100 nucleotides. The capture probe can comprise about 90 nucleotides. The capture probe can comprise about 80 nucleotides. The capture probe can comprise about 70 nucleotides. The capture probe can comprise about 50 nucleotides. The capture probe can comprise about 40 nucleotides. The capture probe can comprise about 30 nucleotides. The capture probe can comprise about 20 nucleotides. The capture probe can comprise about 10 nucleotides. The capture probe can be about 10 to about 400 nucleotides, about 10 to about 200 nucleotides, about 10 to about 120 nucleotides, about 20 to about 120 nucleotides, about 20 to about 60 nucleotides, or about 30 to about 50 nucleotides.

The temperature for ligation of a capture probe to the template nucleic acid can be about 70°C, about 65°C, about 60°C, about 55°C, about 50°C, about 45°C, or about 45°C to about 55°C.

The capture probe can further comprise label. The label can be fluorescent. The fluorescent label can be an organic fluorescent dye, metal chelate, carbon nanotube, quantum dot, gold particle, or fluorescent mineral. The label can be radioactive. The label can be biotin. The capture probe can bind to labeled nucleic acid binder molecule. The nucleic acid binder molecule can be antibody, antibiotic, histone, antibody, or nuclease.

A plurality of capture probes can be used in a sample. The plurality of capture probes can be designed to have similar melting temperatures. The melting temperatures for a set of capture probes can be within about 15°C, within about 10°C, within about 5°C, or within about 2°C. The melting temperature for one or more capture probes can be about 85°C, about 80°C, about 75°C, about 70°C, about 65°C, about 60°C, about 55°C, or about 50°C. The melting temperature for the capture probe can be about 50°C to about 85°C, about 55°C to about 80°C, about 45°C to about 60°C, or about 52°C to about 58°C.

The capture probe can comprise a molecular barcode (MB). The capture probe can comprise and adaptor sequence (e.g., for binding to an adaptor primer). The capture probe can comprise a target specific region (TSR). The capture probe can comprise an index for distinguishing samples. The capture probe can comprise a restriction endonuclease cleavage site, e.g., between the TSR and the adaptor sequence. The molecular barcode or index can be 5' of the adaptor sequence and 5' of the TSR.

### a. Molecular barcodes

Molecular barcodes can provide a unique identification tool and allow for observation of specificity binding between a probe and the template. The molecular barcode can comprise about 25 nucleotides, about 20 nucleotides, about 15 nucleotides, about 10 nucleotides, about 8 nucleotides, or about 4 nucleotides. The molecular barcode can comprise DNA. The molecular barcode can comprise RNA. The molecular barcode can comprise uracil. In some cases, the molecular barcode does not comprise uracil. The capture probe can comprise an index. The index can be used to identify a sample. The index can be 2 to 16 nucleotides in length, or about 6 or about 8 nucleotides.

### b. Adaptor and adaptor primer

The adaptors in the capture probes can provide locations for primer binding for amplification. The adaptor primers can complementarily bind to sites on the adaptors and can be used to amplify the target sequence in the template. The adaptor primers can have a length of about 50 nucleotides. The adaptor primers can have a length of about 45 nucleotides. The adaptor primers can have a length of about 40 nucleotides. The adaptor primers can have a length of about 35 nucleotides. The adaptor primers can have a length of about 30 nucleotides. The adaptor primers can have a length of about 25 nucleotides. The adaptor primers can have a length of about 20 nucleotides. The adaptor primers can have a length of about 15 nucleotides. The adaptor primers can have a length of about 18 to about 30 nucleotides.

The adaptor can comprise DNA or RNA. The adaptor can be naturally occurring or synthetic nucleic acid. The adaptor can have modified heterocyclic bases. The modification can be methylated purines or pyrimidines, acylated purines or pyrimidines, alkylated riboses, or other heterocycles. The adaptor can have modified sugar moieties. The modified sugar moieties can include peptide nucleic acid. The adaptor can comprise peptide nucleic acid. The adaptor can comprise threose nucleic acid. The adaptor can comprise locked nucleic acid. The adaptor can comprise hexitol nucleic acid. The adaptor can comprise flexible nucleic acid. The adaptor can comprise glycerol nucleic acid.

### c. Target specific regions (TSR)

TSR of a capture probe or bridge probe can be designed to hybridize to the target sequence of a template. TSR can be designed against bisulfite converted target sequence or bisulfite protected target sequence. In some cases, 100% of target specific region of probe can be complementary to target sequence of the template. About 75% of the target specific region of probe can be complementary to target sequence of the template. About 50% of the target specific region of probe can be complementary to target sequence of the template. The target specific region can comprise about 35 nucleotides, about 30 nucleotides, about 25 nucleotides, about 20 nucleotides, about 15 nucleotides, about 10 nucleotides, or about 5 nucleotides. The target specific region an anneal to a target sequence described herein.

### X. Adaptors / Adaptor primers / Amplification

Attached, added, or incorporated adaptors can provide sites for primer hybridization for amplification. A first adaptor (AD1) can be attached to the template via a capture probe or an adaptor anchor probe. A primer against AD1 can be utilized to synthesize a strand complementary to the template. A second adaptor (AD2) can be attached to 5' end of template and/or 3' end of the complementary strand to further amplify the template. A library can be constructed using AD1 primer and AD2 primer. Selective amplification can be performed using AD1 primer and primer against TSR or its flanking regions.

The adaptor can be a single-stranded nucleic acid. The adaptor can be double-stranded nucleic acid. The adaptor can be partial duplex, with a long strand longer than a short strand, or with two strands of equal length.

### XI. Bridge probes

Bridge probes are used to hybridize a template nucleic acid molecule with target sequence and an adaptor anchor probe. The bridge probes allow indirect association an adaptor anchor probe and template and thereby facilitating their attachment. The ligation rate of a free adaptor anchor probe and template can be very low because of the randomness of the interaction. But a hybridized bridge probe can increase the probability of ligation between adaptor anchor probe and a template compared to that with a free adaptor anchor probe. The bridge probe can comprise DNA. The bridge probe can comprise of RNA. The bridge probe can comprise of uracil and methylated cytosine. The bridge probe might not comprise of uracil.

The bridge probes comprise target specific region (TSR) that hybridizes to target sequence. The bridge probes comprise adaptor landing sequence (ALS) that hybridizes to bridge binding sequence of adaptor anchor probe. The bridge probe can comprise a linker connecting TSR and ALS. The TSR can be located in the 3'-portion of the bridge probe. The TSR can be located in the 5'-portion of the bridge probe.

The bridge probe can comprise one or more molecular barcodes. The bridge probe can comprise one or more binding moieties. The binding moiety can be a biotin. The binding moieties can be attached to a support. The support can be a bead. The bead can be a streptavidin bead.

The bridge probe can comprise about 400 nucleotides, about 300 nucleotides, about 200 nucleotides, about 120 nucleotides, about 100 nucleotides, about 90 nucleotides, about 80, about 70 nucleotides, about 50 nucleotides, about 40 nucleotides, about 30 nucleotides, about 20 nucleotides, or about 10 nucleotides.

Multiple bridge probes can be used to anneal to multiple target sequences in a sample. The bridge probes can be designed to have similar melting temperatures. The melting temperatures for a set of bridge probes can be within about 15°C, within about 10°C, within about 5°C, or within about 2°C. The melting temperature for one or more bridge probes can be about 75°C, about 70°C, about 65°C, about 60°C, about 55°C, about 50°C, about 45°C, or about 40°C. The melting temperature for the bridge probe can be about 40°C to about 75°C, about 45°C to about 70°C, 45°C to about 60°C, or about 52°C to about 58°C.

Use of an adaptor anchor probe along with one or more bridge probe around a particular bridge probe can help to stabilize the hybridization of the particular bridge probe to the its target sequence through synergistic effect. A hybridization temperature to form the multiple bridge probe assembly can be higher than the melting temperature of a single bridge probe. The higher temperature can result in a better capture specificity by reducing nonspecific hybridization that can occur at lower temperature. The hybridization temperature can be about 5°C, about 10°C, about 15°C, or about 20°C higher than the melting temperature of individual bridge probe. The hybridization temperature can be about 5°C to about 20°C higher than the melting temperature of a bridge probe, or about 5°C to about 20°C higher than an average melting temperature of a plurality of bridge probes.

The hybridization temperature for multiple bridge probes can be about 75°C, about 70°C, about 65°C, about 60°C, about 55°C, or about 50°C. The hybridization temperature for multiple bridge probes can be about 50°C to about 75°C, 55°C to about 75°C, 60°C to about 75°C, or 65°C to about 75°C.

The bridge probe can further comprise a label. The label can be fluorescent. The fluorescent label can be organic fluorescent dye, metal chelate, carbon nanotube, quantum dot, gold particle, or fluorescent mineral. The label can be radioactive. The label can be biotin. The bridge probe can bind to labeled nucleic acid binder molecule. The nucleic acid binder molecule can be antibody, antibiotic, histone, antibody, or nuclease.

The bridge probe can comprise a linker. The linker can comprise about 30 nucleotides, about 25 nucleotides, about 20 nucleotides, about 15 nucleotides, about 10 nucleotides, or about 5 nucleotides. The linker can comprise about 5 to about 20 nucleotides.

The linker can comprise non-nucleic acid polymers (e.g., string of carbons). The linker non-nucleotide polymer can comprise about 30 units, about 25 units, about 20 units, about 15 units, about 10 units, or about 5 units.

The bridge probe can be blocked at the 3' and/or 5' end. The bridge probe can lack a 5' phosphate. The bridge probe can lack a 3' OH. The bridge probe can comprise a 3'ddC, 3'inverted dT, 3'C3 spacer, 3' amino, or 3' phosphorylation.

### XII. Adaptor anchor probe

The adaptor anchor probe can comprise about 400 nucleotides, about 200 nucleotides, about 120 nucleotides, about 100 nucleotides, about 90 nucleotides, about 80 nucleotides, about 70 nucleotides, about 50 nucleotides, about 40 nucleotides, about 30 nucleotides, about 20 nucleotides, or about 10 nucleotides. The adaptor anchor probe can be about 20 to about 70 nucleotides.

The melting temperature of adaptor anchor probe to the bridge probe can be about 65°C, about 60°C, about 55°C, about 50°C, about 45°C. or about 45°C to about 70°C.

The adaptor anchor probe can comprise a label. The label can be fluorescent. The fluorescent label can be an organic fluorescent dye, metal chelate, carbon nanotube, quantum dot, gold particle, or fluorescent mineral. The label can be radioactive. The label can be biotin. The adaptor anchor probe can bind to labeled nucleic acid binder molecule. The nucleic acid binder molecule can be antibody, antibiotic, histone, antibody, or nuclease.

The adaptor anchor probe can comprise a molecular barcode (MB). The adaptor anchor probe can comprise an adaptor sequence (e.g., for binding to an adaptor primer). The capture probe can comprise a bridge binding sequence (BBS). The adaptor anchor probe can comprise from 1to100 BBSs. The adaptor anchor probe can comprise an index for distinguishing samples. The molecular barcode or index can be 5' of the adaptor sequence and 5' of the BBS.

### XIII. Enzymes

Examples of DNA polymerases that can be used in the methods described herein include Klenow polymerase, Bst DNA polymerase, Bca polymerase, phi 29 DNA polymerase, Vent polymerase, Deep Vent polymerase, Taq polymerase, T4 polymerase, T7 polymerase, or E. coli DNA polymerase 1.

Examples of exonucleases that can be used the methods described herein include exonucleases associated with DNA polymerases I (polA), II (polB) and III (dnaQ/mutD), DNA Polymerase I, large (Klenow) fragment, T4 DNA polymerase, Exonuclease I, Exonuclease III, Exonuclease IV, Exonuclease VII, Exonuclease IX, Exonuclease X, RecBCD exonuclease, RecJ exonuclease, RecE exonuclease, SbcCD endo/exonuclease, ribonuclease T, or TatD exonuclease. The exonuclease can be a 3' to 5' exonuclease or a 5' to 3' exonuclease.

Restriction endonucleases that can be used in the methods described herein can be Type 1, Type II, Type III, or Type IV. The restriction endonucleases can include AciI, HindIII, SspI, MluCI, BspMI, EcoP15, EcoRI, HsdR, HsdM, HhaI, NotI, FokI, or BaeI. The restriction endonuclease can be a 4-cutter, 5-cutter, or 6-cutter.

### XIV. Downstream analysis of amplification products

The amplified products generated using methods described herein can be further analyzed using various methods including southern blotting, polymerase chain reaction (PCR) (e.g., real-time PCR (RT-PCR), digital PCR (dPCR), droplet digital PCR (ddPCR), quantitative PCR (Q-PCR), nCounter analysis (Nanostring technology), gel electrophoresis, DNA microarray, mass spectrometry (e.g., tandem mass spectrometry, matrix-assisted laser desorption ionization time of flight mass spectrometry (MALDI-TOF MS), chain termination sequencing (Sanger sequencing), or next generation sequencing.

The next generation sequencing can comprise 454 sequencing (ROCHE) (using pyrosequencing), sequencing using reversible terminator dyes (ILLUMINA sequencing), semiconductor sequencing (THERMOFISHER ION TORRENT), single molecule real time (SMRT) sequencing (PACIFIC BIOSCIENCES), nanopore sequencing (e.g., using technology from OXFORD NANOPORE or GENIA), microdroplet single molecule sequencing using pyrophosphorolyis (BASE4), single molecule electronic detection sequencing, e.g., measuring tunnel current through nanoelectrodes as nucleic acid (DNA/RNA) passes through nanogaps and calculating the current difference (QUANTUM SEQUENCING from QUANTUM BIOSYSTEMS), GenapSys Gene Electornic Nano-Integrated Ultra-Sensitive (GENIUS) technology (GENAPYS), GENEREADER from QIAGEN, sequencing using sequential hybridization and ligation of partially random oligonucleotides with a central determined base (or pair of bases) identified by a specific fluorophore (SOLiD sequencing). The sequencing can be paired-end sequencing.

The number of target sequences from a sample that can be sequenced using methods described herein can be about 5, 10, 15, 25, 50, 100, 1000, 10,000, 100,000, or 1,000,000, or about 5 to about 100, about 100 to about 1000, about 1000 to about 10,000, about 10,000 to about 100,000, or about 100,000 to about 1,000,000.

Nucleic acid libraries generated using methods described herein can be generated from more than one sample. Each library can have a different index associated with the sample. For example, a capture probe or an adaptor anchor probe can comprise an index that can be used to identify nucleic acids as coming from the same sample (e.g., a first set of capture probes or adaptor anchor probes comprising the same first index can be used to generate a first library from a first sample from a first subject, and a second set of capture probes or adaptor anchor probes comprising the same second index can be used to generate a second library from a second sample from a second subject, the first and second library can be pooled, sequenced, and an index can be used to discern from which sample a sequenced nucleic acid was derived). Amplified products generated using the methods described herein can be used to generate libraries from at least 2, 5, 10, 25, 50, 100, 1000, or 10,000 samples, each library with a different index, and the libraries can be pooled and sequenced, e.g., using a next generation sequencing technology.

The sequencing can generate at least 100, 1000, 5000, 10,000, 100,000, 1,000,000, or 10,000,000 sequence reads. The sequencing can generate between about 100 sequence reads to about 1000 sequence reads, between about 1000 sequence reads to about 10,000 sequence reads, between about 10,000 sequence reads to about 100,000 sequence reads, between about 100,000 sequence reads and about 1,000,000 sequence reads, or between about 1,000,000 sequence reads and about 10,000,000 sequence reads.

The depth of sequencing can be about 1x, 5x, 10x, 50x, 100x, 1000x, or 10,000x The depth of sequencing can be between about 1x and about 10x, between about 10x and about 100x, between about 100x and about 1000x, or between about 1000x and about 10000x.

### XV. Applications

### a. Detection of nucleic acid features

The amplified nucleic acid products generated using the methods described herein can be analyzed for one or more nucleic acid features. The one or more nucleic acid features can be one or more methylation events. The methylation can be methylation of a cytosine in a CpG dinucleotide. The methylated base can be a 5-methylcytosine. A cytosine in a non-CpG context can be methylated. The methylated or unmethylated cytosines can be in a CpG island. A CpG island can be a region of a genome with a high frequency of CpG sites. The CpG island can be at least 200 bp, or about 300 to about 3000 bp. The CpG island can be a CpG dinucleotide content of at least 60%. The CpG island can be in a promoter region of a gene. The methylation can be 5-hmC (5-hydroxymethylcytosine), 5-fC (5-formylcytosine), or 5-caC (5-carboxylcytosine). The methods described herein can be used to detect methylation patterns, e.g., of DNA from a solid tissue or from a biological fluid, e.g., plasma, serum, urine, or saliva comprising, e.g., cell-free DNA.

The one or more nucleic acid features can be a de novo mutation, nonsense mutation, missense mutation, silent mutation, frameshift mutation, insertion, substitution, point mutation, single nucleotide polymorphism (SNP), single nucleotide variant (SNV), de novo single nucleotide variant, deletion, rearrangement, amplification, chromosomal translocation, interstitial deletion, chromosomal inversion, loss of heterozygosity, loss of function, gain of function, dominant negative, or lethal mutation. The amplified nucleic acid products can be analyzed to detect a germline mutation or a somatic mutation. The one or more nucleic acid features can be associated with a condition, e.g., cancer, autoimmune disease, neurological disease, infection (e.g., viral infection), or metabolic disease.

### b. Diagnosis/detections/monitoring

The disclosed methods can also be used to diagnosis or detect a condition. The condition can be a psychological disorder. The condition can be aging. The condition can be a disease. The condition (e.g., disease) can be a cancer, a neurological disease (e.g., Alzheimer's disease, autism spectrum disorder, Rett Syndrome, schizophrenia), immunodeficiency, skin disease, autoimmune disease (e.g., Ocular Behcet's disease, systemic lupus erythematosus (SLE), rheumatoid arthritis (RA), multiple sclerosis, infection (e.g., viral infection), or metabolic disease (e.g., hyperglycemia, hyperlipidemia, type 2 diabetes mellitus). The cancer can be, e.g., colon cancer, breast cancer, liver cancer, bladder cancer, Wilms cancer, ovarian cancer, esophageal cancer, prostate cancer, bone cancer, or hepatocellular carcinoma, glioblastoma, breast cancer, squamous cell lung cancer, thyroid carcinoma, or leukemia (see e.g., Jin and Liu (2018) DNA methylation in human disease. Genes & Diseases, 5:1-8). The condition can be Beckwith-Wiedemann Syndrome, Prader-Willi syndrome, or Angelman syndrome.

The methylation patterns of cell-free DNA generated using methods provided herein can be used as markers of cancer (see e.g., Hao et al., DNA methylation markers for diagnosis and prognosis of common cancers. Proc. Natl. Acad. Sci. 2017; international PCT application publication no. WO2015116837). The methylation patterns of cell-free DNA can be used to determine tissues of origin of DNA (see e.g., international PCT application publication no. WO2005019477). The methods described herein can be used to determine methylation haplotype information and can be used to determine tissue or cell origin of cell-free DNA (see e.g., Seioighe et al, (2018) DNA methylation haplotypes as cancer markers. Nature Genetics 50, 1062-1063; international PCT application publication no. WO2015116837; U.S. patent application publication no. 20170121767). The methods described herein can be used to detect methylation levels, e.g., of cell-free DNA, in subjects with cancer and subjects without cancer (see e.g., Vidal et al. A DNA methylation map of human cancer at single base-pair resolution. Oncogenomics 36, 5648-5657; international PCT application publication no. WO2014043763). The methods described herein can be used to determine methylation levels or to determine fractional contributions of different tissues to a cell-free DNA mixture (see e.g., international PCT application publication no. WO2016008451). The methods described herein can be used for tissue of origin of cell-free DNA, e.g., in plasma, e.g., based on comparing patterns and abundance of methylation haplotypes (see e.g., Tang et al., (2018) Tumor origin detection with tissue-specific miRNA and DNA methylation markers. Bioinformatics 34, 398-406; international PCT application publication no. WO2018119216). The methods described herein can be used to distinguish cancer cells from normal cells and to classify different cancer types according to their tissues of origin (see e.g., U.S. Patent Application Publication No. 20170175205A1). The methods provided herein can be used to detect fetal DNA or fetal abnormalities using a maternal sample (see e.g., Poon et al. (2002) Differential DNA Methylation between Fetus and Mother as a Strategy for Detecting Fetal DNA in Maternal Plasma. Clinical Chemistry, 48: 35-41).

The disclosed methods can be used for monitoring of a condition. The condition can be disease. The disease can be a cancer, a neurological disease (e.g., Alzheimer's disease), immunodeficiency, skin disease, autoimmune disease (e.g., Ocular Behcet's disease), infection (e.g., viral infection), or metabolic disease. The cancer can be in remission. Since the disclosed methods can use cfDNA and ctDNA to detect low level of abnormalities, the present disclosure can provide relatively noninvasive method of monitoring diseases. The disclosed methods can be used for monitoring a treatment or therapy. The treatment or therapy can be used for a condition, e.g., a disease, e.g., cancer, or for any condition disclosed herein.

### EXAMPLES

### Example 1

### Ligation of adaptor anchor probe

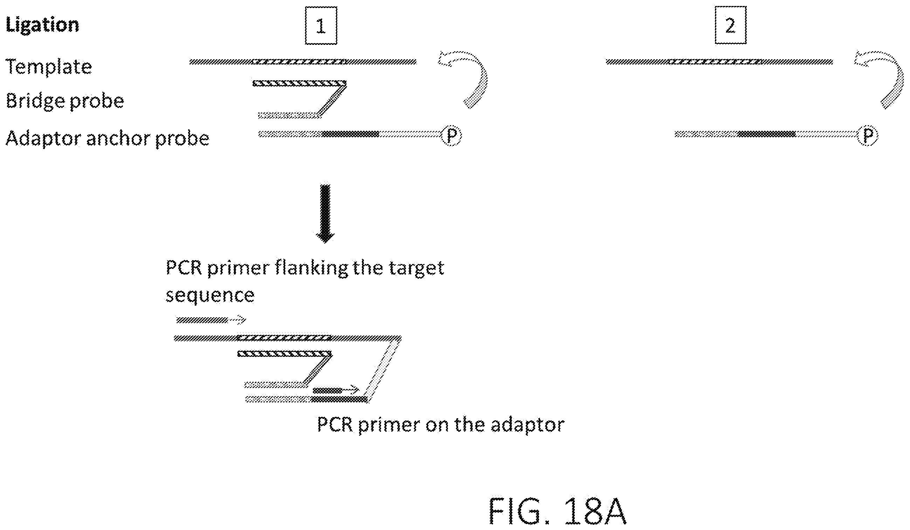

**FIG. 18A** provides a schematic overview of two reaction schemes. In the reaction scheme on the left (1), bridge probe, template, and adaptor anchor probe are incubated together. The bridge probe is hybridized to a target sequence on the template, and the bridge probe is hybridized to an adaptor landing sequence on the adaptor anchor probe (1; top). The hybridization facilitates proximity ligation of the phosphorylated 5' end of the adaptor anchor probe and the 3' end of the template (1; top). Then, PCR is used to amplify the ligation product (1; bottom). In a second reaction on the right (2), template and adaptor anchor probe are incubated together in an absence of a bridge probe (2; top). Similar reaction steps are performed as in reaction scheme (1).

In this example, in reaction scheme (1), an adaptor anchor probe with target specific binding sequence (SEQ ID: 2) was ligated to a template DNA with a known target sequence (SEQ ID: 1) facilitated by hybridization of a bridge probe (SEQ ID: 3). The bridge probe comprised a target specific region that was designed to be complementary to the target sequence of the template. The italicized letters in SEQ ID: 1 and SEQ ID: 2 (TABLE 1) represent the target sequence and the target specific region of the template and the bridge probe, respectively. The bridge probe further comprised an adaptor landing sequence that was complementary to the bridge binding sequence of the bridge probe and an oligo-T linker connecting the target specific region and the adaptor landing sequence. The bridge binding sequence and the adaptor landing sequence are underlined in SEQ ID: 2 and SEQ ID: 3 in TABLE 1. In addition to the bridge binding sequence, the adaptor anchor probe was designed to contain an adaptor and a molecular barcode of a unique sequence. The bolded sequences in SEQ ID: 2 represent the AD1 primer binding site. Primers that anneal to AD1 and TSR were used to amplify the target sequence of the template. "N" represents any of the four DNA nucleotides. Different 3' ends of template were tested to find which nucleotide is preferentially ligated and sequenced. The four nucleotides at 5' end of adaptor anchor probe represent a molecular barcode. Various combinations of molecular barcodes were synthesized and mixed with the template.

**TABLE 1: Sequence Listings**

| **SEQ ID NO:** | **Type** | **Sequences** |
|---|---|---|
| 1 | Template | 5- CGTCGCTATCAAGGAATTAAGAGAAGCAACATCTCCG AAAGCCAACAAGGAAATCCTCGATGTGAGTTTN -3′ |
| 2 | Adaptor anchor probe | 5- GNNNGATCGTCGGACTGTAGAACTCTGAACCGAA CGACGACATAGACCAC-3′ |
| 3 | Bridge probe | 5- GTGGTCTATGTCGTCGTTCGTTTTTTTTTTTTTTTCTTG TTGGCTTTCGGAGATGTTGC-3′ |
| 4 | AD1 primer | GTTCAGAGTTCTACAGTCCGACGATC |
| 5 | TSR primer | CGTCGCTATCAAGGAATTAAG |

The following are the hybridization and ligation conditions for reaction scheme (1). 1fmole of template, 10fmole of bridge probe, and 200fmole of adaptor anchor probe were mixed in 12ul of volume and denatured at 95°C for 30 second then placed on ice. 2uL of 10x CircLigase buffer, 4uL of 5M Betaine, 1µL of 50 mM MnCl₂ were added to the denatured DNA mix to reach 2.5mM MnCl₂, 1M Betaine, 0.033M Tris-acetate (pH 7.5), 0.066 M potassium acetate, and 0.5 mM DTT final concentration in 20uL of final volume. Hybridization was performed by incubation of the mixture at 50°C for 30 min on a MJ thermocycler. Ligation was done by adding 100 unit of Circligase II to the mixture and incubating for 50°C for 20 min on a MJ thermocycler. 1 µL of the ligated product was taken as template for PCR reaction with 0.2µM of PCR primers. 20 cycles of PCR with denaturation condition of 95°C for 15 sec, annealing condition at 58°C for 15 sec, and extension condition at 68°C for 30 sec were conducted. The conditions for reaction scheme (2) were the same, except that the bridge probe was not included in the mix of the template and adaptor anchor probe. 10µL of PCR product was run on 2% agarose gel at 80V for 20min and the DNA bands were visualized on a UV light box with the gel stained by 1X GelRed.

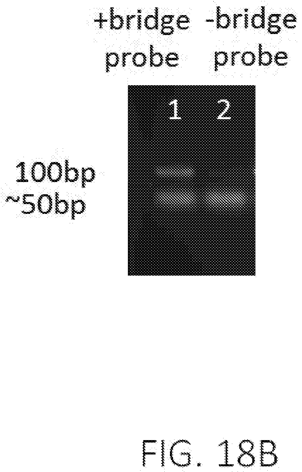

As shown in **FIG. 18B****,** two bands appeared at around 100 bp and 50 bp regions in Lane 1, which held the PCR reaction products of reaction scheme (1). 100 bp is consistent with the size of ligated template and adaptor anchor probe, suggesting the ligation was successful. The lower band is the expected size of the primers used in the amplification step. No ~100 bp band is detectable in Lane 2 for reaction scheme (2), suggesting that the bridge probe in reaction scheme (1) facilitated ligation of the template and adaptor anchor probe.

### Example 2

### Adaptor Addition by Exonuclease Digestion and Extension

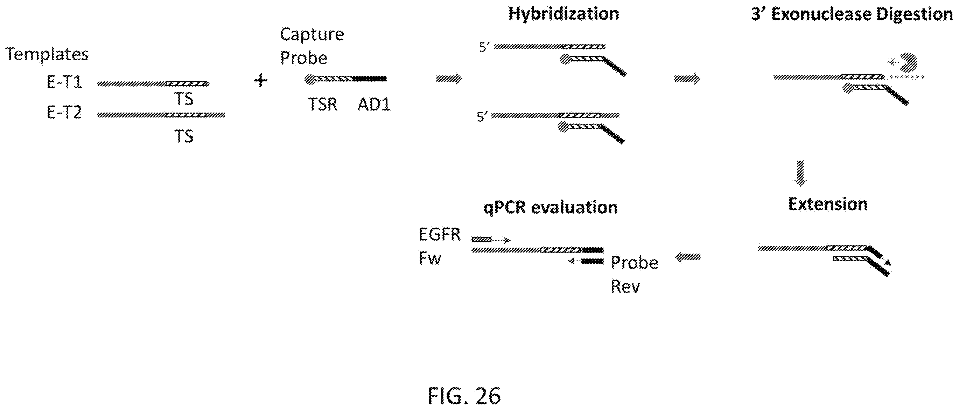

FIG. 26 provides a schematic overview of the capture probe hybridization and digestion by 3' to 5' exonuclease. For the hybridization capture, the capture probes were hybridized to a target sequence on the templates (E-T1, SEQ ID NO. 6 and E-T2, SEQ ID NO. 7). For the reaction, the synthetic template E-T1(Input Mixtures 1,3,5,7 of Table 3) or E-T2 (Input Mixtures 2,4,6,8 of Table 3) was mixed with capture probe (SEQ ID NO. 8) at a ratio of 1:1 in concentration (1 uM) in Duplex buffer. The DNA mixtures were denatured at 92°C for 2 min, and allowed to cool down to room temperature on a thermo cycler.

Table 2 lists the sequences of the template nucleic acids, the capture probe, PCR primers used, as well as the extension product from the experiment. Adaptor sequences are indicated in bold and the target sequences are italicized. E-T2 template comprised more nucleotide sequences down-stream of the target specific region (TSR) compared to E-T1, such that when hybridized to the capture probe, E-T2 had single-stranded 3' overhang.

**TABLE 2: Sequence Listings**

| **SEQ ID NO:** | **Type** | **Sequences** |
|---|---|---|
| 6 | Template (E-T1) | 5- CCCGTCGCTATCAAGGAATTAAGAGAAGCAACATCT GTGGGGGTCCATGGCTCTGAACCT -3′ |
| 7 | Template (E-T2) | 5- CCCGTCGCTATCAAGGAATTAAGAGAAGCAACATCT GTGGGGGTCCATGGCTCTGAACCTCAGGCCCACCTTTT CTTCCGGTT -3′ |
| 8 | Capture probe | 5- GACTGGAGTTCAGACGTGTGCTCTTCCGATCTAG GTTCAGAGCCATGGACCCCCACATTTTTTTTTT-3′ |
| 9 | EGFR forward primer | CCCGTCGCTATCAAGGAATTAAGA |
| 10 | Capture probe reverse primer | GACTGGAGTTCAGACGTGTGC |
| 11 | Extension Product | CCCGTCGCTATCAAGGAATTAAGAGAAGCAACATCT GTGGGGGTCCATGGCTCTGAACCTAGATCGGAAGAG CACACGTCTGAACTCCAGTC |

After hybridization, the 3'to 5' exonuclease was used to digest the single-stranded 3' overhang on the template. For exonuclease digestion and extension, the DNA mixtures were reacted with either DNA Polymerase I, large (Klenow) fragment or T4 polymerase. Table 3 lists the combinations of the template and enzyme used for each mixture.

**TABLE 3: Input Mixtures and Experimental Results**

| **Input Mix** | **1** | **2** | **3** | **4** | **5** | **6** | **7** | **8** |
|---|---|---|---|---|---|---|---|---|
| Template | E-T1 | E-T2 | E-T1 | E-T2 | E-T1 | E-T2 | E-T1 | E-T2 |
| Enzyme | Klenow | | | | T4 Pol | | | |
| Enzyme | + | + | - | - | + | + | - | - |
| qPCR (Ct) | 29.96 | 30.34 | N/D | N/D | 29.78 | 30.18 | N/D | N/D |
| Adaptor addition result | + | + | - | - | + | + | - | - |

About 12000 copies of E-T1/E-T2 and capture probe-annealed product were used for each digestion and extension mixture. For Klenow reaction mixture, 1X NEB buffer 2, 50uM dNTPs, and 0.1U/ul Klenow were added and incubated at 25°C for 15 min. As for T4 Polymerase mixture, 1X NEB buffer 2.1, 10uM dNTPs, and 0.06U/ul T4 polymerase were added and incubated at 12°C for 15 min.

The PCR products were evaluated by qPCR using PCR primers (EGFR forward primer, SEQ ID NO. 9 and capture probe reverse primer, SEQ ID NO. 10). The PCR products that were not reacted with Klenow or T4 polymerase (Input Mix 3,4,7, and 8) and whose templates thereby still retain the 3' overhang were not detected to comprise the adaptor (see Table 3). In contrast, adaptor additions were detected in Input Mix 1, 2, 5, and 6. The products of Input Mix 1, 2, 5, and 6 were also detected to have the sequences of SEQ ID NO. 10. The cycle threshold (Ct) values with E-T1 template were lower than those with E-T2 regardless of the exonuclease/polymerase used, indicating the extension occurred more readily with E-T1 template since E-T1 did not comprise 3' overhang.

### Example 3

### Adaptor Addition by Exonuclease Digestion and Extension Using T4 Polymerase

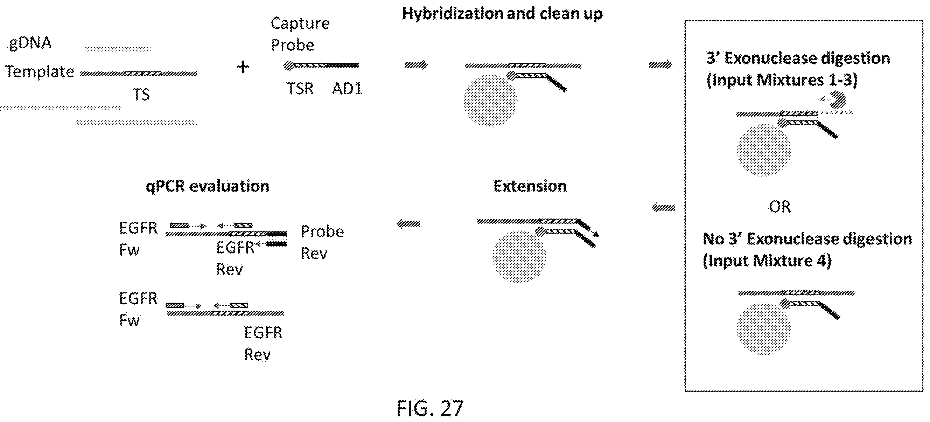

FIG. 27 provides a schematic overview of the capture probe hybridization and digestion by 3' to 5' exonuclease. The capture probe was hybridized with a target sequence on the template. A fragmented genomic DNA (gDNA), known to comprise SEQ ID NO. 12 was used as the template. For the hybridization capture, 20ng of fragmented (peak size 160bp) template gDNA was used with 10 pmol of capture probes. The DNA input and hybridization probes were denatured in hybridization buffer at 95°C for 30 secs, and the mixture was allowed to gradually cool down to 60°C. The hybridization was incubated at 60°C for 1 hour on a thermo cycler. The final hybridization buffer contained 100ng/ul of DNA, 1ug/ul Bovine Serum Albumin (BSA), 1ug/ul Ficoll, 1ug/ul Polyvinylpyrrolidone (PVP), 0.075M sodium citrate, 0.75 M NaCl, 5x SSC, and 1X Denhardt's solutions.

To cleanup, the hybridized assemblies were incubated with streptavidin beads (Thermo Fisher Dynabeads M270 Streptavidin) at room temperature for 10 min. The cleanup was performed with three washes (wash 1 :5X SSPE, 1%SDS; wash 2: 2X SSPE, 0.1%; wash 3: 0.1X SSPE, 0.01% triton).

Then, a 3' to 5' exonuclease (T4 polymerase) was added to the capture assemblies to digest the single-stranded 3' overhang on the templates and to extend the template. The capture assemblies were resuspended in 25 ul T4 polymerase mix (Input Mix 1, 2, 3), and incubated at 12°C for 15 min. Reaction was stopped by a heat kill at 95°C for 1 min. Input Mix 4 was not treated with T4 polymerase.

qPCR was conducted to evaluate the addition of the adaptor. The enriched DNA was evaluated by qPCR using probes EGFR targeting sequence (EGFR forward primer, SEQ ID NO. 13 and reverse primer, SEQ ID NO. 14). The adaptor addition is evaluated by EGFR and adaptor sequence PCR (EGFR forward primer, SEQ ID NO. 13 and probe reverse primer, SEQ ID NO. 15, see Tables 4 and 5). FIG. 27 illustrates the schematic for qPCR evaluation. Adaptor addition was detected in all samples (Input Mix 1, 2, and 3) treated with T4 polymerase but not in the sample with no T4 polymerase reaction (see Table 5). The cycling threshold (Ct) values of the mixtures (Input Mix 1, 2, and 3) that were subjected to exonuclease digestion were lower than that of non-digested template (Input Mix 4). In all the samples, the Ct for EGFR PCR were similar, indicating the EGFR was captured in all reactions, and the Ct for the enriched DNA was compared to the same portion of gDNA without capture enrichment and it was found that 65% of EGFR was recovered.

**TABLE 4: Sequence Listing**

| **SEQ ID NO:** | **Type** | **Sequences** |
|---|---|---|
| 12 | Template [partial] | 5’- TGTGGGGGTCCATGGCTCTGAACCTCAGGCCCACCT-3’ |
| 13 | EGFR forward primer | 5-CCCGTCGCTATCAAGGAATTAAGA -3' |
| 14 | EGFR reverse primer | 5-CCACACAGCAAAGCAGAAACTCAC-3' |
| 15 | Probe reverse primer | 5-GACTGGAGTTCAGACGTGTGC-3' |

**TABLE 5: Input Mixtures and Experimental Results**

| **Input Mix** | **1** | **2** | **3** | **4** |
|---|---|---|---|---|
| Template | gDNA | gDNA | gDNA | gDNA |
| T4 Pol | + | + | + | - |
| qPCR (ct) EGFR Fw/Rev | 32.29 | 31.78 | 31.7 | 32.78 |
| qPCR (ct) EGFR Fw/Probe Rev | 33.05 | 32.2 | 32.48 | 38.3 |
| Adaptor addition result | + | + | + | - |

## Claims

1. A method comprising:
(a) hybridizing a first target specific region of a first bridge probe to a first target sequence of a template nucleic acid molecule, wherein the first bridge probe comprises an adaptor sequence positioned 5' of the target specific region and hybridizing a first landing sequence of the first bridge probe to a first bridge binding sequence of an anchor probe;
(b) hybridizing a second target specific region of a second bridge probe to a second target sequence of the template nucleic acid molecule, and hybridizing a second landing sequence of the second bridge probe to a second bridge binding sequence of the anchor probe, thereby forming a complex comprising the template nucleic acid molecule, the first bridge probe, the second bridge probe, and the anchor probe,
wherein the anchor probe is not attached to a solid support during (a) and (b);
(c) following (a) and (b), coupling the anchor probe to the solid support; and
(d) following (c), contacting the complex with a 3' to 5' exonuclease, wherein the 3' to 5' exonuclease cleaves the 3' end of the template nucleic acid molecule, and extending the 3' end of the template nucleic acid molecule using the adaptor sequence as a template, thereby generating a first extension product.

2. The method of claim 1, wherein the anchor probe comprises a binding moiety, and wherein the coupling comprises attaching the binding moiety to the solid support.

3. The method of claim 1 or 2, wherein the solid support is a bead.

4. The method of claim 3, wherein the bead is a streptavidin bead.

5. The method of any one of claims 2-4, wherein the binding moiety is a biotin.

6. The method of claim 1, wherein the template nucleic acid molecule is cell-free DNA.

7. The method of claim 1, further comprising contacting the first extension product with a primer comprising a sequence of the adaptor to generate a second extension product.

8. The method of claim 7, further comprising i) hybridizing a target specific primer to the second extension product and extending the target specific primer to generate a third extension product or ii) attaching a second double-stranded adaptor to the 5' end of the first extension product and the 3' end of the second extension product.

9. The method of any one of claims 1-8, further comprising detecting a methylation pattern of the template nucleic acid molecule.

10. The method of any one of claims 1-9, further comprising analyzing the amplified nucleic acid product for one or more nucleic acid features of the template nucleic acid molecule selected from the group of the following features: a de novo mutation, nonsense mutation, missense mutation, silent mutation, frameshift mutation, insertion, substitution, point mutation, single nucleotide polymorphism (SNP), single nucleotide variant (SNV), de novo single nucleotide variant, deletion, rearrangement, amplification, chromosomal translocation, interstitial deletion, chromosomal inversion, loss of heterozygosity, loss of function, gain of function, dominant negative, or lethal mutation.

## Patentansprüche

1. Verfahren, umfassend:
(a) Hybridisieren einer ersten zielspezifischen Region einer ersten Brückensonde an eine erste Zielsequenz eines Nukleinsäure-Matrizenmoleküls, wobei die erste Brückensonde eine Adaptersequenz umfasst, welche 5' der zielspezifischen Region positioniert ist, und Hybridisieren einer ersten Landesequenz der ersten Brückensonde an eine erste Brückenbindungssequenz einer Ankersonde,
(b) Hybridisieren einer zweiten zielspezifischen Region einer zweiten Brückensonde an eine zweite Zielsequenz des Nukleinsäure-Matrizenmoleküls und Hybridisieren einer zweiten Landesequenz der zweiten Brückensonde an eine zweite Brückenbindungssequenz der Ankersonde, wodurch ein Komplex gebildet wird, welcher das Nukleinsäure-Matrizenmolekül, die erste Brückensonde, die zweite Brückensonde und die Ankersonde umfasst,
wobei die Ankersonde während (a) und (b) nicht an einen festen Träger gebunden ist,
(c) Koppeln der Ankersonde an den festen Träger nach (a) und (b) und
(d) In-Kontakt-Bringen des Komplexes mit einer 3'- bis 5'-Exonuklease nach (c), wobei die 3'- bis 5'-Exonuklease das 3'-Ende des Nukleinsäure-Matrizenmoleküls spaltet, und Verlängern des 3'-Endes des Nukleinsäure-Matrizenmoleküls unter Verwendung der Adaptersequenz als Matrize, wodurch ein erstes Verlängerungsprodukt erzeugt wird.

2. Verfahren nach Anspruch 1, wobei die Ankersonde eine Bindungsgruppe umfasst und wobei das Koppeln das Anbringen der Bindungsgruppe an den festen Träger umfasst.

3. Verfahren nach Anspruch 1 oder 2, wobei der feste Träger ein Kügelchen ist.

4. Verfahren nach Anspruch 3, wobei das Kügelchen ein Streptavidin-Kügelchen ist.

5. Verfahren nach einem der Ansprüche 2 bis 4, wobei die Bindungsgruppe ein Biotin ist.

6. Verfahren nach Anspruch 1, wobei das Nukleinsäure-Matrizenmolekül zellfreie DNA ist.

7. Verfahren nach Anspruch 1, ferner umfassend das In-Kontakt-Bringen des ersten Verlängerungsprodukts mit einem Primer, der eine Sequenz des Adapters umfasst, um ein zweites Verlängerungsprodukt zu erzeugen.

8. Verfahren nach Anspruch 7, das ferner umfasst: i) Hybridisieren eines zielspezifischen Primers mit dem zweiten Verlängerungsprodukt und Verlängern des zielspezifischen Primers, um ein drittes Verlängerungsprodukt zu erzeugen, oder ii) Anbringen eines zweiten doppelsträngigen Adapters an das 5'-Ende des ersten Verlängerungsprodukts und das 3'-Ende des zweiten Verlängerungsprodukts.

9. Verfahren nach einem der Ansprüche 1 bis 8, das ferner umfasst: Nachweisen eines Methylierungsmusters des Nukleinsäure-Matrizenmoleküls.

10. Verfahren nach einem der Ansprüche 1 bis 9, welches ferner umfasst: Analysieren des amplifizierten Nukleinsäureprodukts auf ein oder mehrere Nukleinsäuremerkmale des Nukleinsäure-Matrizenmoleküls, ausgewählt aus der Gruppe der folgenden Merkmale: eine De-novo-Mutation, eine Nonsense-Mutation, eine Missense-Mutation, eine stille Mutation, eine Mutation mit einer Verschiebung des Leserasters, eine Insertion, eine Substitution, eine Punktmutation, ein Einzelnukleotid-Polymorphismus (SNP), eine Einzelnukleotid-Variante (SNV), eine De-novo-Einzelnukleotid-Variante, eine Deletion, eine Umlagerung, eine Amplifikation, eine chromosomale Translokation, eine interstitielle Deletion, eine chromosomale Inversion, ein Verlust der Heterozygotie, ein Funktionsverlust, ein Funktionsgewinn, eine dominante Negativmutation oder eine letale Mutation.

## Revendications

1. Procédé comprenant :
(a) l'hybridation d'une première région spécifique cible d'une première sonde passerelle à une première séquence cible d'une molécule d'acide nucléique matrice, dans lequel la première sonde passerelle comprend une séquence adaptatrice positionnée du côté 5' de la région spécifique cible et l'hybridation d'une première séquence d'amarrage de la première sonde passerelle à une première séquence de liaison de passerelle d'une sonde d'ancrage ;
(b) l'hybridation d'une seconde région spécifique cible d'une seconde sonde passerelle à une seconde séquence cible de la molécule d'acide nucléique matrice, et l'hybridation d'une seconde séquence d'amarrage de la seconde sonde passerelle à une seconde séquence de liaison de passerelle de la sonde d'ancrage, ce qui permet de former un complexe comprenant la molécule d'acide nucléique matrice, la première sonde passerelle, la seconde sonde passerelle et la sonde d'ancrage,
dans lequel la sonde d'ancrage n'est pas fixée à un support solide pendant (a) et (b) ;
(c) à la suite de (a) et (b), le couplage de la sonde d'ancrage au support solide ; et
(d) à la suite de (c), la mise en contact du complexe avec une exonucléase 3' -> 5', dans lequel l'exonucléase 3' -> 5' clive l'extrémité 3' de la molécule d'acide nucléique matrice, et l'extension de l'extrémité 3' de la molécule d'acide nucléique matrice en utilisant la séquence adaptatrice en tant que matrice, ce qui permet de générer un premier produit d'extension.

2. Procédé selon la revendication 1, dans lequel la sonde d'ancrage comprend un fragment de liaison, et dans lequel le couplage comprend la fixation du fragment de liaison au support solide.

3. Procédé selon la revendication 1 ou 2, dans lequel le support solide est une bille.

4. Procédé selon la revendication 3, dans lequel la bille est une bille de streptavidine.

5. Procédé selon l'une quelconque des revendications 2 à 4, dans lequel le fragment de liaison est une biotine.

6. Procédé selon la revendication 1, dans lequel la molécule d'acide nucléique matrice est de l'ADN acellulaire.

7. Procédé selon la revendication 1, comprenant en outre la mise en contact du premier produit d'extension avec une amorce comprenant une séquence de l'adaptateur afin de générer un deuxième produit d'extension.

8. Procédé selon la revendication 7, comprenant en outre i) l'hybridation d'une amorce spécifique pour une cible au deuxième produit d'extension et l'extension de l'amorce spécifique pour la cible afin de générer un troisième produit d'extension, ou ii) la fixation d'un deuxième adaptateur double brin à l'extrémité 5' du premier produit d'extension et à l'extrémité 3' du deuxième produit d'extension.

9. Procédé selon l'une quelconque des revendications 1 à 8, comprenant en outre la détection d'un motif de méthylation de la molécule d'acide nucléique matrice.

10. Procédé selon l'une quelconque des revendications 1 à 9, comprenant en outre l'analyse du produit d'acide nucléique amplifié pour rechercher une ou plusieurs caractéristiques d'acide nucléique de la molécule d'acide nucléique matrice choisies dans le groupe des caractéristiques suivantes : mutation de novo, mutation non-sens, mutation faux sens, mutation silencieuse, décalage du cadre de lecture, insertion, substitution, mutation ponctuelle, polymorphisme nucléotidique unique (SNP), variant mononucléotidique (SNV), variant mononucléotidique de novo, délétion, réarrangement, amplification, translocation chromosomique, délétion interstitielle, inversion chromosomique, perte d'hétérozygotie, perte de fonction, gain de fonction, dominante négative, ou mutation létale.
